# EUROPEAN PATENT SPECIFICATION

(11) **EP 2 614 778 B1**
(45) Date of publication and mention of the grant of the patent: **29.03.2017**
(21) Application number: 12198192.2
(22) Date of filing: 19.12.2012
(51) Int. Cl.: A61B 10/02, A61B 10/04, A61M 5/32, A61B 17/3205, A61B 50/36

(54) **Tissue harvesting apparatus**
Gewebeentnahmevorrichtung
Appareil de récolte de tissus

(30) Priority: 13.01.2012 JP 2012005384
(43) Date of publication of application: 17.07.2013
(73) Proprietor: Fujifilm Corporation, Minato-ku Tokyo 106-8620 (JP)
(72) Inventor: Tanaka, Toshizumi, Kanagawa, 258-8538 (JP); Itoh, Koji, Kanagawa, 258-8538 (JP); Izaki, Toshihiko, Kanagawa, 258-8538 (JP); Iyama, Shozo, Kanagawa, 258-8538 (JP)
(74) Representative: Klunker . Schmitt-Nilson . Hirsch

(56) References cited:
- WO-A1-2010/024950
- JP-A- 2000 232 983
- US-A1- 2010 312 141
- US-B1- 6 537 266

## Description

### BACKGROUND OF THE INVENTION

### 1. Field of the Invention

The present invention relates to a tissue harvesting apparatus according to claim 1, and in particular to a tissue harvesting apparatus which is used to perform harvesting of a living body tissue.

### 2. Description of the Related Art

As a procedure when harvesting a living body tissue (cells) of an internal organ such as the liver or the pancreas, the following is known. First, an ultrasonic endoscope is inserted into the body cavity, thereby making a leading end portion thereof be located at the stomach or the duodenum. Then, a puncture needle of a tissue harvesting apparatus is inserted into a treatment tool insertion channel of the ultrasonic endoscope, thereby making the puncture needle be led out of a lead-out port of the leading end portion of the ultrasonic endoscope. Subsequently, the puncture needle is punctured into a target internal organ while viewing an ultrasonic image, whereby cells taken into the puncture needle or an internal tube are harvested.

Further, as tissue harvesting apparatuses in the related art, those as disclosed in JP3661470B and JP4468664B are also known.

A tissue harvesting apparatus of JP3661470B is constituted by a catheter which is inserted into a treatment tool insertion channel of an ultrasonic endoscope, as an insertion unit, and the catheter is configured of a sheath member (an external tube) which is arranged at an outermost periphery, a puncture pipe body which is slidably inserted and arranged in the inside of the sheath member, and a cutter pipe body (an internal tube) which is slidably inserted and arranged in the inside of the puncture pipe body. A needle tip obliquely cut is formed at a leading end portion of the puncture pipe body and a suction hole is formed on the base end side of the puncture pipe body. An annular blade edge, the outer peripheral edge side of which is sharpened in a blade shape, is formed at a leading end of the cutter pipe body. Then, the puncture pipe body is made so as to move in an axial direction along with the cutter pipe body in the inside of the sheath member by a given operation of an operating unit provided at a base end portion of the catheter and the cutter pipe body is made so as to move in the axial direction in the inside of the puncture pipe body by another given operation of the operating unit.

According to this, the puncture pipe body may be punctured into a target internal organ by making the needle tip of the puncture pipe body be led out of a leading end opening of the sheath member. Then, the cells are suctioned into the inside through the suction hole of the puncture pipe body by creating a state where the blade edge of the cutter pipe body has been attracted further to the base end side than the suction hole of the puncture pipe body and making the inside of the puncture pipe body be under negative pressure by a suction operation of a syringe mounted on the operating unit. The cells suctioned into the inside through the suction hole are cut out by pushing out the blade edge of the cutter pipe body so as to pass the suction hole of the puncture pipe body in this state, and the cut out cells are taken into the inside of the cutter pipe body.

A tissue harvesting apparatus (a puncture needle) of JP4468664B is provided with an insertion unit which is inserted into a treatment tool insertion channel of an ultrasonic endoscope, and the insertion unit is constituted by a sheath (an external tube) which is arranged at an outermost periphery, a needle tube which is slidably inserted and arranged in the inside of the sheath, and a stylet which is slidably inserted and arranged in the inside of the needle tube. A needle tip obliquely cut is formed at a leading end portion of the needle tube. In this tissue harvesting apparatus, by making the needle tip of the needle tube be led out of the sheath, thereby puncturing the needle tube into a target internal organ, cells are taken into the inside of the needle tube. The stylet is used by being inserted into the inside of the needle tube when taking out the cells taken into the inside of the needle tube to the outside.
US 2010/312141 A1 discloses a tissue harvesting apparatus according to the preamble of claim 1, having a needle tube with a slanted tip at a leading end thereof. The needle tube may be drawn back into an external tube so that the slanted tip does not protrude from the leading end of the external tube. An operating unit comprises an annular finger grip.

### SUMMARY OF THE INVENTION

The tissue harvesting apparatuses as disclosed in JP3661470B and JP4468664B are normally for single use (disposable), and if harvesting of a living body tissue is finished, the tissue harvesting apparatus is disposed. At the time of disposal, the insertion unit (the catheter) is wound in the form of a loop and then housed in an housing bag such as a plastic bag.

On the other hand, the external tube (the sheath member or the sheath) constituting the insertion unit of the tissue harvesting apparatus is a close-contact spring or a tube made of resin or the like, and the puncture pipe body (the needle tube), the cutter pipe body (the internal tube), and the stylet which are inserted into the inside of the external tube are made of metal. Then, since the insertion unit which is constituted by these is inserted into the treatment tool insertion channel, the insertion unit has flexibility and also a property of resilience to bending.

For this reason, there is a concern that when disposing the insertion unit of the tissue harvesting apparatus with it wound in the form of a loop, winding in the form of a loop may be released due to the resilience property of the insertion unit or released in the housing bag. Since the puncture pipe body (the needle tube) is present in the inside of the insertion unit, in a case where the above-described situation occurs, there is a concern that a worker may be stabbed with the needle tip of the puncture pipe body.

The present invention has been made in view of the above-mentioned problems and an object of the present invention is to provide a tissue harvesting apparatus which can reliably prevent a worker from being stabbed with a needle tip of a needle tube at the time of disposal.

In order to achieve the above-described object, according to an aspect of the present invention, there is provided a tissue harvesting apparatus including: a needle tube having a needle tip at a leading end thereof, the needle tip performing puncture in order to harvest a cellular tissue in the body cavity; an external tube in which the needle tube is inserted into inside and the needle tip of the needle tube moves in and out from a leading end; an operating unit which is provided at a base end of the external tube and moves the needle tube in an axial direction; and a needle housing hole which is arranged in the operating unit and is configured to house and retain the needle tip of the needle tubeinside of the needle housing hole.

According to the above aspect of the present invention, by housing the needle tip of the needle tube in the needle housing hole when disposing the tissue harvesting apparatus, the tissue harvesting apparatus can be disposed without exposing the needle tip of the needle tube, and thus a contingency situation in which a worker who performs disposal work is stabbed with the needle of the needle tube can be reliably prevented.

In the tissue harvesting apparatus related to the above aspect of the present invention, a come off prevention member fixedly supporting the needle tube housed in the needle housing hole may be arranged in the inside of the needle housing hole. According to this, a worker being stabbed with the needle tip of the needle tube can be more reliably prevented.

An aspect in which the come off prevention member is a pressing member pressing the needle tube housed in the needle housing hole to a side wall surface of the needle housing hole, an aspect in which the come off prevention member is a convex member holding the needle tube housed in the needle housing hole between the convex member and a side wall surface of the needle housing hole, or an aspect in which the come off prevention member is an elastic member in which a hole housing the needle tip is drilled by the needle tip when housing the needle tip of the needle tube in the needle housing hole is possible.

In the tissue harvesting apparatus related to the above aspect of the present invention, an aspect is possible in which the operating unit has a locking groove locking the external tube wound in the form of a loop along with the needle tube when disposing the tissue harvesting apparatus. According to this, since winding of the external tube is not easily released, a situation in which the needle tube housed in the needle housing hole comes out of the needle housing hole also does not easily occur, and therefore, a worker being stabbed with the needle of the needle tube can be more reliably prevented.

In the tissue harvesting apparatus related to the above aspect of the present invention, an aspect is possible in which the needle housing hole is formed in an operating rod which is arranged at a rearmost end of the operating unit. In a case where the tissue harvesting apparatus includes an insertion member which is inserted into the inside of the needle tube, an aspect may be considered in which the operating rod which is arranged at the rearmost end and in which the needle housing hole is formed is an operating rod moving the insertion member in the axial direction with respect to the needle tube.

In the tissue harvesting apparatus related to the above aspect of the present invention, an aspect is possible in which the needle housing hole and the locking groove are formed in an operating rod which is arranged at a rearmost end of the operating unit. In a case where the tissue harvesting apparatus further includes an insertion member which is inserted into the inside of the needle tube, an aspect is considered in which the operating rod which is arranged at the rearmost end and in which the needle housing hole and the locking groove are formed is an operating rod moving the insertion member in the axial direction with respect to the needle tube.

In the tissue harvesting apparatus related to another aspect of the present invention, an aspect is possible in which the needle housing hole is formed in a mounting member that is detachably mounted in the operating unit. Further, in the tissue harvesting apparatus related to the above aspect of the present invention, an aspect is possible in which the needle housing hole and the locking groove are formed in a mounting member that is attachable and detachable in the operating unit. That is, by forming the needle housing hole or the locking groove in a mounting member that is attachable and detachable in the operating unit, rather than the needle housing hole or the locking groove being directly formed in the operating unit, even in a case where the operating unit is small and thus there is no space to provide the needle housing hole or the locking groove, the needle housing hole and the locking groove can be arranged in the operating unit.

According to the present invention, it is possible to reliably prevent a worker from being stabbed with a needle tip of a needle tube of a tissue harvesting apparatus at the time of disposal.

### BRIEF DESCRIPTION OF THE DRAWINGS

Fig. 1 is a cross-sectional view of a main section showing a state where a tissue harvesting apparatus related to an embodiment of the present invention is incorporated in an ultrasonic endoscope.
Fig. 2 is the overall configuration diagram of a first embodiment of the tissue harvesting apparatus shown in Fig. 1.
Fig. 3 is a cross-sectional view of a leading end portion of a catheter constituting the tissue harvesting apparatus of the first embodiment.
Fig. 4 is a cross-sectional view of the tissue harvesting apparatus of the first embodiment.
Fig. 5 is an explanatory diagram of an operation of the tissue harvesting apparatus of the first embodiment and a diagram showing a state just before the catheter is stuck into the body.
Fig. 6 is an explanatory diagram of an operation in a state where in the tissue harvesting apparatus of the first embodiment, a leading end of a puncture pipe body of the catheter is punctured into a living body tissue harvesting place.
Fig. 7 is the overall configuration diagram of a second embodiment of the tissue harvesting apparatus shown in Fig. 1.
Fig. 8 is a cross-sectional view of a leading end portion of a catheter constituting the tissue harvesting apparatus of the second embodiment.
Fig. 9 is a cross-sectional view of the tissue harvesting apparatus of the second embodiment.
Fig. 10 is an explanatory diagram of an operation of the tissue harvesting apparatus of the second embodiment and a diagram showing a state just before the catheter is stuck into the body.
Fig. 11 is an explanatory diagram of an operation in a state where in the tissue harvesting apparatus of the second embodiment, a leading end of a puncture pipe body of the catheter performs suction of a living body tissue at a living body tissue harvesting place.
Fig. 12 is an explanatory diagram of an operation showing a state where in the tissue harvesting apparatus of the second embodiment, the living body tissue taken into the puncture pipe body is cut by a cutter pipe body.
Fig. 13 is an explanatory diagram of an operation showing a state where in the tissue harvesting apparatus of the second embodiment, transfer of the recovered tissue is performed.
Fig. 14 is a cross-sectional view of a main section showing another example of the puncture pipe body.
Fig. 15 is a perspective view showing the configuration of a needle-stick prevention mechanism with an operating rod of an operating unit of the tissue harvesting apparatus enlarged.
Fig. 16 is a perspective view showing a state where a sheath member is wound using the needle-stick prevention mechanism at the time of disposal.
Fig. 17 is an enlarged perspective view showing a locking groove.
Figs. 18A and 18B are cross-sectional views showing the configuration of come off prevention means of a first embodiment in the inside of a needle housing hole.
Fig. 19 is a plan view showing the configuration of come off prevention means of a second embodiment in the inside of the needle housing hole.
Fig. 20 is a cross-sectional view showing the configuration of come off prevention means of a third embodiment in the inside of the needle housing hole.
Fig. 21 is a perspective view of an operating rod showing another form to replace the locking groove.
Fig. 22 is a perspective view of an operating rod showing another form to replace the locking groove.
Fig. 23 is a perspective view showing a first embodiment of a needle-stick prevention tool.
Fig. 24 is a perspective view showing the first embodiment of the needle-stick prevention tool.
Fig. 25 is a perspective view showing a second embodiment of the needle-stick prevention tool.
Fig. 26 is a perspective view showing a third embodiment of the needle-stick prevention tool.
Fig. 27 is a plan view (a cross-sectional view) showing the third embodiment of the needle-stick prevention tool.

### DESCRIPTION OF THE PREFERRED EMBODIMENTS

Hereinafter, embodiments for carrying out the present invention will be described.

A tissue harvesting apparatus for puncture with the present invention applied thereto will be described as being constructed such that the tissue harvesting apparatus is inserted into the body cavity through a treatment tool insertion channel formed in an ultrasonic endoscope that performs electronic convex scanning. However, as guide means of the tissue harvesting apparatus, an ultrasonic endoscope of a scanning method other than this, a treatment tool insertion channel of a normal endoscope which is not provided with an ultrasonic diagnostic mechanism, a trocar, or the like can be adopted as the guide means, and in a case where the tissue harvesting apparatus is inserted into the trocar, the entirety of the tissue harvesting apparatus can also be constituted by hard members.

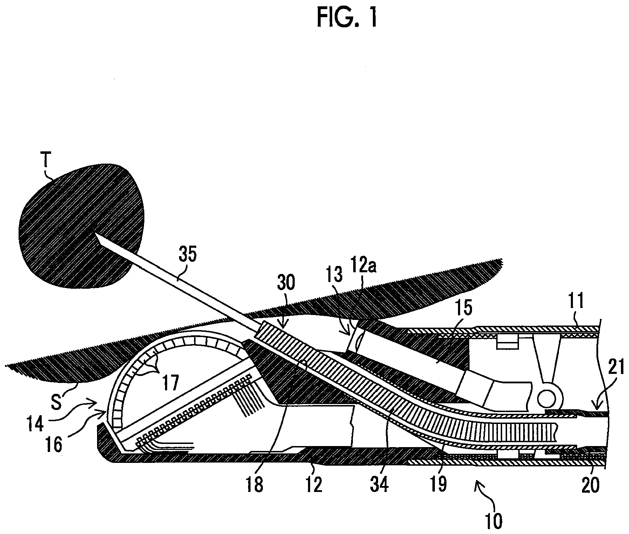

First, the configuration of a leading end portion of the ultrasonic endoscope that guides the tissue harvesting apparatus is shown in Fig. 1. In the drawing, reference numeral 10 denotes an insertion section into the body cavity. The insertion section 10 is constituted by consecutively connecting a leading end portion main body 12 to a leading end of an angle section 11, and in the leading end portion main body 12, an endoscopic observation section 13 is provided on the base end side and an ultrasonic observation section 14 is provided on the leading end side. The endoscopic observation section 13 is provided at an inclined portion 12a on the base end side of the leading end portion main body 12 and constituted such that an observation visual field is directed obliquely forward.

In Fig. 1, an illumination mechanism 15 provided with a light guide constituting the endoscopic observation section 13 is shown, and an observation mechanism is provided along with the illumination mechanism 15. However, illustration of the observation mechanism is omitted. In addition, as the observation mechanism, a solid-state image sensing device is used or an image guide is used.

The ultrasonic observation section 14 includes an ultrasonic transducer unit 16 mounted in an opening portion 12b provided at a leading end of the leading end portion main body 12. The ultrasonic transducer unit 16 is for performing electronic convex scanning and is constituted by arranging a large number of strip-shaped ultrasonic vibrators 17 in an arc shape.

A treatment tool lead-out portion 18 is formed at a position between the endoscopic observation section 13 and the ultrasonic observation section 14. The treatment tool lead-out portion 18 is a pathway having a predetermined inner diameter that is perforated in the leading end portion main body 12, and a connection pipe 19 is connected to the treatment tool lead-out portion 18. The connection pipe 19 is bent at a predetermined angle and a flexible tube 20 is connected to a base end portion thereof. Therefore, a treatment tool insertion channel 21 is constituted by the treatment tool lead-out portion 18, the connection pipe 19, and the flexible tube 20, the treatment tool lead-out portion 18 extends forward obliquely with respect to an axis line of the insertion section 10, the flexible tube 20 extends in an axial direction of the insertion section 10, and an intermediate portion of the connection pipe 19 is bent by a predetermined angle.

Reference numeral 30 denotes a tissue harvesting apparatus, and the tissue harvesting apparatus 30 is made so as to be able to be inserted into the treatment tool insertion channel 21, thereby appearing and disappearing from the treatment tool lead-out portion 18. Then, by bringing the leading end portion main body 12 into contact with a body cavity inner wall S, putting a tissue harvesting place T in an ultrasonic observation visual field by the ultrasonic observation section 14, sticking a leading end of the tissue harvesting apparatus 30 from the treatment tool lead-out portion 18 into the body cavity inner wall S, and leading the leading end to the tissue harvesting place T, a living body tissue, that is, cells of the tissue harvesting place can be harvested. Then, the tissue harvesting apparatus 30 is drawn out of the treatment tool insertion channel 21 and the harvested tissue is transferred to a container containing formalin solution or the like.

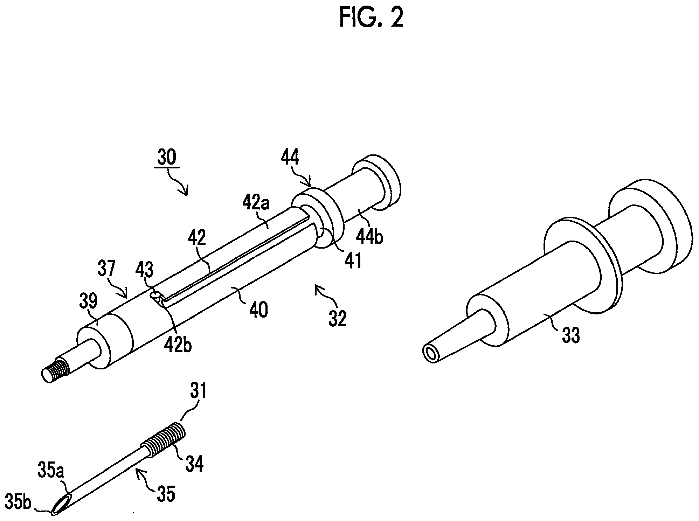

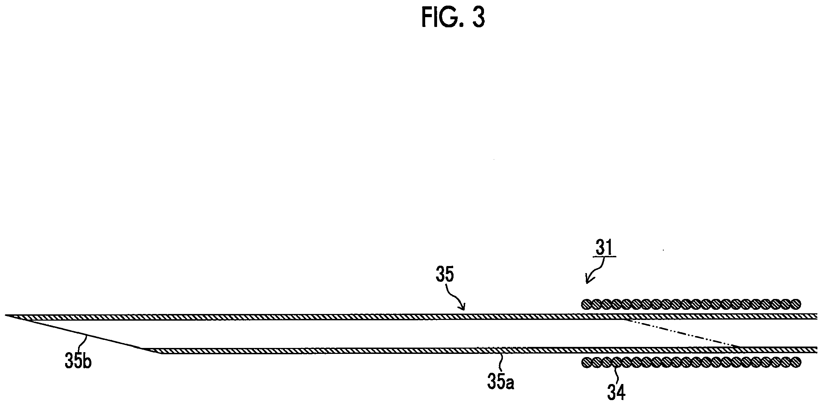

The overall configuration diagram of a first embodiment of the tissue harvesting apparatus 30 is shown in Fig. 2. As is apparent from the same drawing, the tissue harvesting apparatus 30 of the first embodiment includes a catheter 31 and an operating unit 32, and a syringe 33 is detachably connected to a base end portion of the operating unit 32. The catheter 31 is an insertion unit of the tissue harvesting apparatus 30 which is inserted into the treatment tool insertion channel 21, is longer than at least the entire length of the treatment tool insertion channel 21, and is constituted by a double tube member, as shown in Fig. 3. That is, the catheter 31 is constituted by an outer sheath member (an external tube) 34 and a puncture pipe body (a needle tube) 35 inserted into the sheath member 34.

The sheath member 34 is a tubular member having flexibility and can also be formed of a flexible tube or the like. However, in the illustrated example, the sheath member is constituted by a close-contact coil. Further, in the puncture pipe body 35, a needle tip 35b having a pointed leading end is formed by opening a leading end of a thin-walled pipe-shaped main body pipe 35a having an opened leading end, and also obliquely cutting a leading end portion thereof.

Since the puncture pipe body 35 is stuck into the body, at least a leading end portion that includes the needle tip 35b should be hard, and therefore, it is formed of a hard member.

Here, since the catheter 31 is inserted into the treatment tool insertion channel 21, the catheter 31 should have flexibility in a bending direction in order to be able to pass through the bent connection pipe 19 and be smoothly inserted into the angle section 11 even in a state where the angle section 11 is curved. For this purpose, a portion other than the leading end portion that includes the needle tip 35b of the puncture pipe body 35 may also be formed of a tube having flexibility, thereby having a configuration in which the tube and the hard pipe are connected. However, although the puncture pipe body 35 may be formed of a pipe member made of metal or the like over the entire length, because the puncture pipe body 35 has a small diameter and the wall thickness thereof may be made as thin as possible, the puncture pipe body 35 becomes bendable.

The puncture pipe body 35 is made to be movable in a front-and-back direction in the sheath member 34 and moves between a retreated position (a position shown by an imaginary line in Fig. 3) where the needle tip 35b thereof is covered by the sheath member 34 and a working position (a position shown by a solid line in the same drawing) where the needle tip 35b protrudes by a predetermined length from a leading end of the sheath member 34.

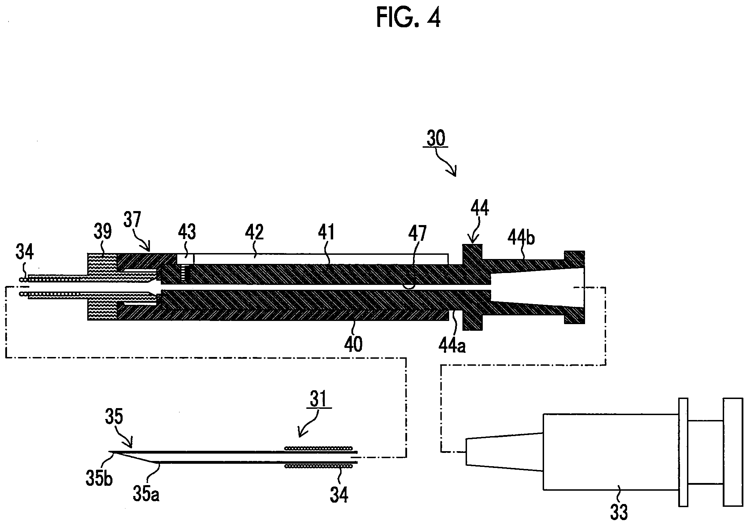

For this purpose, a base end portion of the sheath member 34 is connected to operating means 37 of the operating unit 32 and the puncture pipe body 35 is made so as to appear and disappear from a leading end of the sheath member 34 by the operating means 37. Further, a base end portion of the puncture pipe body 35 is connected to a connecting member 44 (hereinafter referred to as an operating rod 44) of the operating unit 32, and the puncture pipe body 35 slides in the sheath member 34 due to the operating rod 44. The operating unit 32 is constituted by the operating means 37 and the operating rod 44 and the specific configuration thereof is made as shown in Fig. 4.

The base end portion of the sheath member 34 is provided to be fixedly attached to a connecting member 39 and the connecting member 39 is connected to a casing 40 constituting the operating means 37. The casing 40 is constituted by a cylindrical member having a predetermined length, and in the inside thereof, a slider 41 provided to extend as a portion of the operating rod 44 is inserted so as to be able to slide in an axial direction of the casing 40. The slider 41 is hollow and the base end portion of the puncture pipe body 35 is fixedly attached to the inside thereof. Therefore, if the slider 41 (the operating rod 44) is operated so as to be pushed and pulled, the needle tip 35b of the leading end of the puncture pipe body 35 moves in and out from the leading end of the sheath member 34.

Further, a guide hole 42 having a predetermined length is provided in an axial direction in a peripheral body portion of the casing 40 and a pin 43 which is inserted into the guide hole 42 is attached to the slider 41. A leading end of the guide hole 42 is bent at about 90°, thereby becoming a positioning hole portion 42b directed in a circumferential direction. A rear end of the guide hole 42 is provided to extend to a rear end of the casing 40.

A state where the slider 41 is drawn out of the casing 40 and thus the puncture pipe body 35 is drawn into the sheath member 34 is a retreated position of the puncture pipe body 35, and if the transition to a state where the pin 43 is extracted from the rear end of the guide hole 42 and then brought into contact with, for example, the rear end (a portion where the guide hole 42 is not provided) of the casing 40 is made, the puncture pipe body 35 can be retained at the retreated position. At the retreated position, the needle tip 35b of the puncture pipe body 35 is located slightly inside the leading end of the sheath member 34, and thus a state where the needle tip 35b is completely covered by the sheath member 34 is created, whereby at the time of insertion into the treatment tool insertion channel 21, or the like, the needle tip 35b does not stab another object or is not caught on another object, and thus safety is secured and an insertion operation into the treatment tool insertion channel 21 can also be smoothly performed. In addition, a state where the slider 41 is drawn further out of the casing 40 than a state where the pin 43 comes into contact with the rear end of the casing 40, that is, a state where the pin 43 is not engaged with the guide hole 42 becomes a state where the puncture pipe body 35 is completely covered by the sheath member 34, and the position of the puncture pipe body 35 in the state also becomes the retreated position.

On the other hand, if the pin 43 is engaged with the guide hole 42 and the slider 41 is then pushed into the casing 40, the puncture pipe body 35 is led out of the sheath member 34. Then, if the pin 43 is arranged at a position where the pin 43 is engaged with the positioning hole portion 42b of the leading end of the guide hole 42, the puncture pipe body 35 protrudes by a predetermined length from the sheath member 34. This is a working position where puncture can be performed, and a protruding length at this working position becomes the maximum stab length into the body.

Here, the maximum stab length of the puncture pipe body 35 is a length in which the puncture pipe body 35 is stabbed to a position where the sheath member 34 comes into contact with the body cavity inner wall and, also in this state, there is a need to make the needle tip 35b enter the ultrasonic observation visual field by the ultrasonic transducer unit 16. Therefore, the maximum stab length of the puncture pipe body 35 is regulated to the ultrasonic observation visual field.

In order to shift the pin 43 which is provided on the slider 41 side, from the guide hole 42 of the casing 40 to the positioning hole portion 42b at the above-described working position and then stabilize the pin 43 at that position, it is only necessary to relatively turn the casing 40 and the slider 41 (the operating rod 44). In addition, if the width of a shift portion from the guide hole 42 to the positioning hole portion 42b is made slightly narrower than the outer diameter dimension of the pin 43, a click feeling can be obtained at the time of the shift and also the pin 43 can be stably retained. In order to stabilize the slider 41 at the above-described retreated position, it is only necessary to move the pin 43 to a position retreated from an axis line of the guide hole 42, by relatively turning the casing 40 and the slider 41 (the operating rod 44) such that the pin 43 deviated from the guide hole 42 is not easily engaged with the guide hole 42. In addition, if the width of the vicinity of the rear end of the guide hole 42 is made slightly narrower than the outer diameter dimension of the pin 43, a click feeling can be obtained at the time of the shift of the slider 41 to the retreated position, and even if the pin 43 is not retreated from the axis line of the guide hole 42, the slider 41 can be stably retained at the retreated position.

The puncture pipe body 35 also functions as a fluid pathway. This fluid pathway acts as a suction pathway for making negative pressure act and a pathway in which liquid such as formalin solution is pumped so as to discharge a tissue housed in the puncture pipe body 35. Then, as an extending portion of the fluid pathway of the puncture pipe body 35, a flow path 47 is formed in the operating rod 44. Abase end portion of the operating rod 44 becomes a luer lock portion 44b to which the syringe 33 for suction or for liquid pumping is detachably connected.

In addition, a needle-stick prevention mechanism for reliably preventing a worker from being stabbed with the needle tip 35b of the puncture pipe body 35 when disposing the tissue harvesting apparatus 30 after use is provided at the operating rod 44 of the operating unit 32. The details of the needle-stick prevention mechanism will be described later.

This embodiment is constituted as described above, and next, a method of harvesting a tissue in the body by using the tissue harvesting apparatus 30 will be described.

First, the leading end portion main body 12 of the ultrasonic endoscope is arranged at a predetermined position with respect to the body cavity inner wall S. In this state, if a tissue harvesting place in the body is captured in the observation visual field of the ultrasonic transducer unit 16 constituting the ultrasonic observation section 14, the catheter 31 is inserted into the treatment tool insertion channel 21 such that the leading end portion thereof is located in the vicinity of the leading end of the treatment tool lead-out portion 18. Further, the syringe 33 remains connected to the luer lock portion 44b constituting the operating rod 44. However, as the syringe 33, a syringe for suction is used.

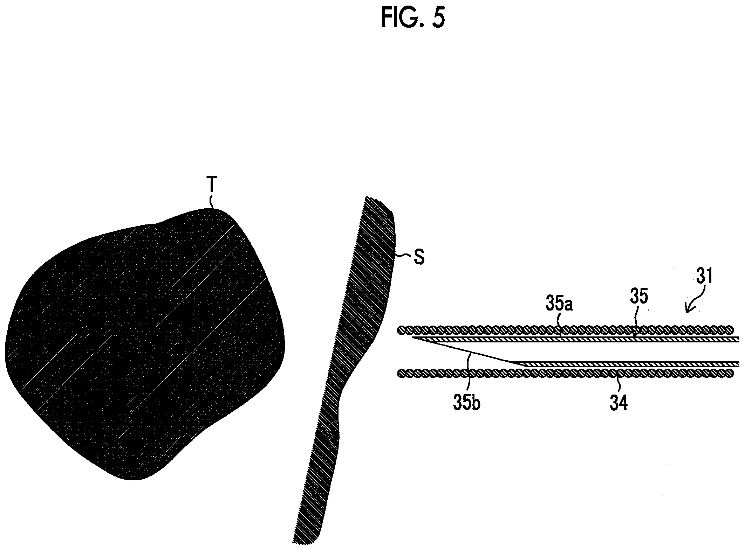

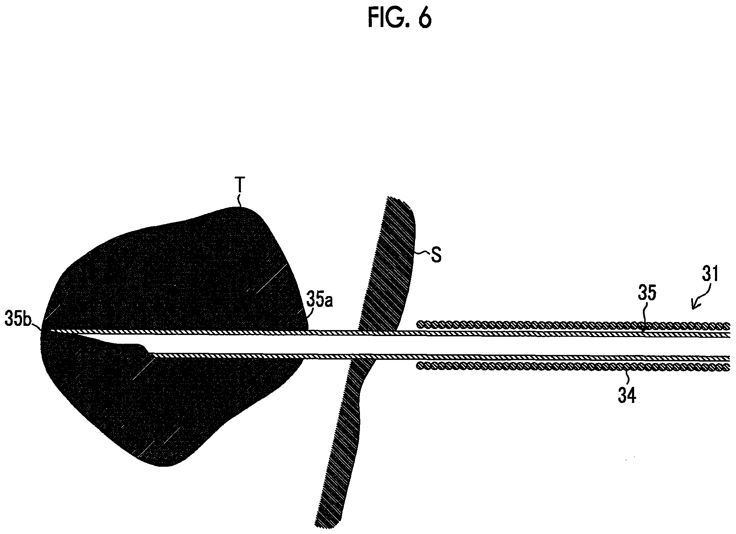

Here, as shown in Fig. 5, the leading end portion of the puncture pipe body 35 in the catheter 31 is covered by the sheath member 34, and in this state, the slider 41 in the operating means 37 is operated by an operation of the operating rod 44 so as to be pushed into the casing 40, whereby the puncture pipe body 35 is led out of the sheath member 34. In this way, as shown in Fig. 6, the needle tip 35b of the leading end of the puncture pipe body 35 is stuck into the body from the body cavity inner wall S.

Since a sticking route of the puncture pipe body 35 into the body can be captured in the ultrasonic observation visual field, the sticking operation can be safely performed and it is possible to reliably target the tissue harvesting place T.

If the needle tip 35b of the puncture pipe body 35 advances, thereby entering the living body tissue harvesting place T, the syringe 33 is operated, thereby making the inside of the puncture pipe body 35 be under negative pressure. Due to the action of the negative pressure, the living body tissue enters the puncture pipe body 35 through the leading end opening portion of the puncture pipe body 35, and thus the living body tissue is harvested into the puncture pipe body 35. Then, in a state where the negative pressure has been applied, the catheter 31 is extracted from the treatment tool insertion channel 21. In addition, an operation when harvesting the living body tissue into the puncture pipe body 35 is not limited thereto. For example, an operation is also possible in which in a state where the needle tip 35b of the puncture pipe body 35 has entered the living body tissue harvesting place T, the inside of the puncture pipe body 35 is repeatedly switched between negative pressure and positive pressure by the syringe 33 and, at that time, an operation such as changing a direction of the needle tip 35b of the puncture pipe body 35 is performed, thereby reliably harvesting the living body tissue into the puncture pipe body 35.

If the living body tissue is harvested by the catheter 31 of the tissue harvesting apparatus 30 in this way and the catheter 31 is then extracted from the treatment tool insertion channel 21, instead of the syringe for suction, a syringe that pumps, for example, formalin solution may be connected to the luer lock portion 44b and the formalin solution pumped from the syringe into a cutter pipe body 36. In this way, the harvested tissue can be transferred to a test tube or the like.

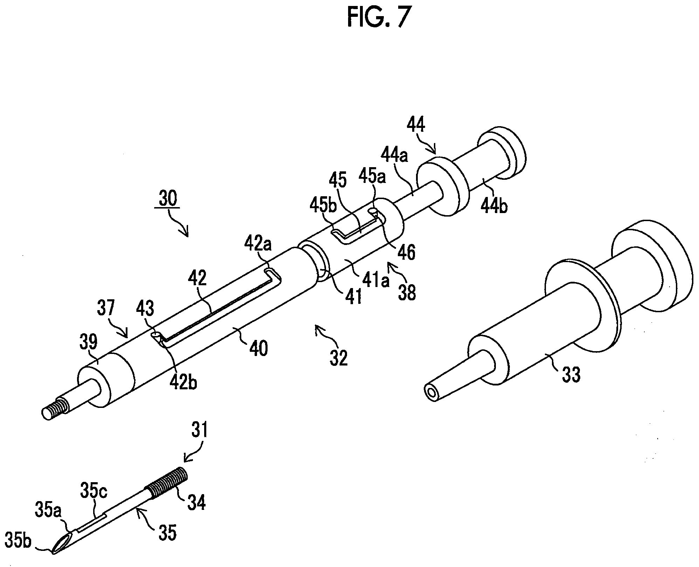

Next, the overall configuration of a second embodiment of the tissue harvesting apparatus 30 is shown in Fig. 7. In addition, constituent elements identical or similar to those of the tissue harvesting apparatus 30 of the first embodiment described in Figs. 2 to 6 are denoted by the same reference numerals and description thereof is partially omitted.

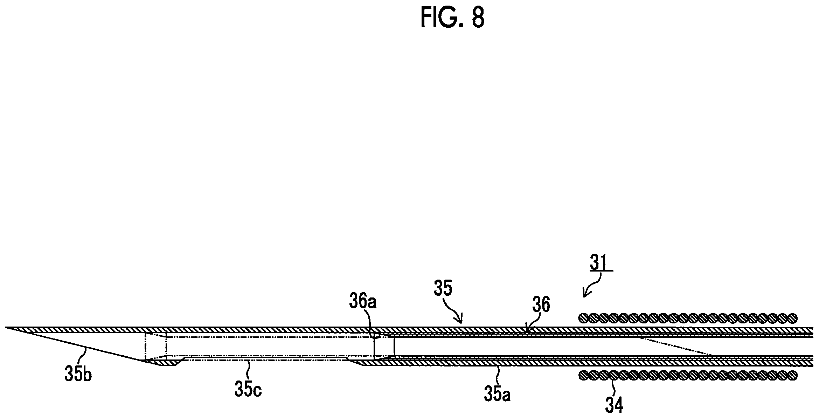

As is apparent from the same drawing, the tissue harvesting apparatus 30 of the second embodiment includes the catheter 31 and the operating unit 32, similarly to the first embodiment, and the syringe 33 is detachably connected to the base end portion of the operating unit 32. On the other hand, the catheter 31 is different from that in the first embodiment and constituted by a triple tube member in which a cutter pipe body 36 (an insertion member) is inserted and arranged in the inside of the puncture pipe body 35, as shown in Fig. 8. That is, the catheter 31 is constituted by the sheath member 34, the puncture pipe body 35, and the cutter pipe body 36 from the outermost circumference side. Further, the operating unit 32 is also different from that in the first embodiment and has second operating means 38 provided between first operating means 37 corresponding to the operating means 37 in the first embodiment and the operating rod 44.

In the catheter 31, a suction hole 35c is perforated at a position close to the base end side of a face obliquely cut as the needle tip 35b, in the side surface of the main body pipe 35a of the puncture pipe body 35.

The cutter pipe body 36 is of the form of a thin-walled pipe in which a substantially entire circumference is inserted into the puncture pipe body 35 so as to slide, and the outer peripheral edge side of a leading end thereof is in a state where it is sharpened in a blade shape, thereby becoming an annular blade edge 36a.

In addition, since the puncture pipe body 35 is stuck into the body, at least the leading end portion that includes the needle tip 35b should be hard, and a leading end portion forming the blade edge 36a of the cutter pipe body 36 is also formed of a hard member so as to fully demonstrate a function as a cutter.

Here, since the catheter 31 is inserted into the treatment tool insertion channel 21, the catheter 31 should have flexibility in a bending direction in order to be able to pass through the bent connection pipe 19 and be smoothly inserted into the angle section 11 even in a state where the angle section 11 is curved. For this purpose, each of a portion other than the leading end portion that includes the needle tip 35b of the puncture pipe body 35 and a portion other than the leading end portion that includes the blade edge 36a of the cutter pipe body 36 may also be formed of a tube having flexibility, thereby having a configuration in which the tube and the hard pipe are connected. However, although each of the puncture pipe body 35 and the cutter pipe body 36 may be formed of a pipe member made of metal or the like over the entire length, because the puncture pipe body 35 and the cutter pipe body 36 have small diameters and the wall thickness thereof may be made as thin as possible, the puncture pipe body 35 and the cutter pipe body 36 becomes bendable.

The puncture pipe body 35 is made to be movable in a front-and-back direction in the sheath member 34 and the cutter pipe body 36 is made so as to move in the front-and-back direction in a state where there is almost no gap with respect to the puncture pipe body 35. The puncture pipe body 35 moves between the retreated position (a position shown by an imaginary line in Fig. 8) where the needle tip 35b thereof is covered by the sheath member 34 and the working position (a position shown by a solid line in the same drawing) where the needle tip 35b protrudes by a predetermined length from the leading end of the sheath member 34. Further, the cutter pipe body 36 is displaced back and forth in the puncture pipe body 35 between a drawn-in position where the blade edge 36a thereof is located further to the base end side than the suction hole 35c and a pushed-out position where the blade edge 36a has passed the suction hole 35c. However, even when the cutter pipe body 36 has been displaced to the pushed-out position, the blade edge 36a of the cutter pipe body 36 is retained at a position further toward the front than a site in the puncture pipe body 35 where the needle tip 35b is formed.

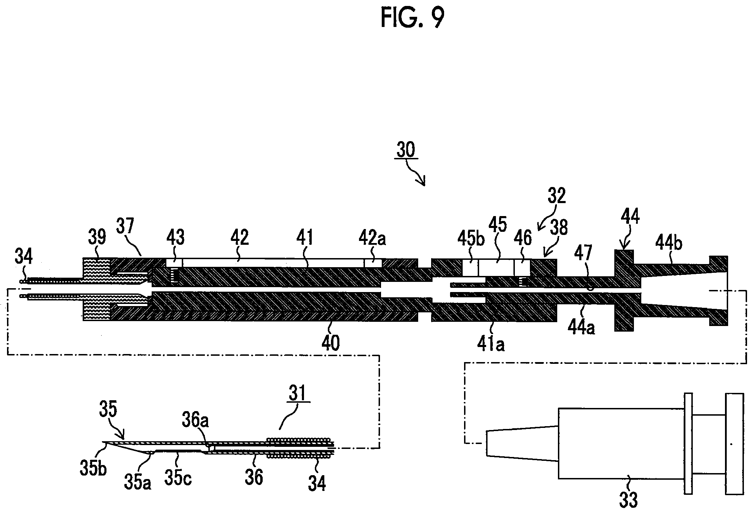

For this purpose, the base end portion of the sheath member 34 is connected to the first operating means 37, whereby the puncture pipe body 35 and the cutter pipe body 36 are made so as to integrally appear and disappear from the leading end of the sheath member 34 by the first operating means 37. Further, the base end portion of the puncture pipe body 35 is connected to the second operating means 38 and the cutter pipe body 36 slides in the puncture pipe body 35 due to the second operating means 38. The operating unit 32 is constituted by the first and second operating means 37 and 38 and the operating rod 44 and the specific configuration thereof is made as shown in Fig. 9.

In addition, in the first embodiment, a member to perform a push-pull operation of the puncture pipe body 35 with respect to the sheath member 34 is the operating rod 44, whereas in the second embodiment, the member is the second operating means 38. Therefore, essentially, the second operating means 38 in the second embodiment has a similar action to the operating rod 44 in the first embodiment. However, because of similarities of a shape and disposition, an operating member which is arranged at the rearmost end of the operating unit 32 in the second embodiment as a constituent element identical or similar to the operating rod 44 in the first embodiment is set to be the operating rod 44 and denoted by the same reference numeral as that in the first embodiment.

The base end portion of the sheath member 34 is provided to be fixedly attached to the connecting member 39 and the connecting member 39 is connected to the casing 40 constituting the first operating means 37. The casing 40 is constituted by a cylindrical member having a predetermined length, and in the inside thereof, the slider 41 is inserted so as to be able to slide in the axial direction of the casing 40. Similarly to the first embodiment, the slider 41 is hollow and the base end portion of the puncture pipe body 35 is fixedly attached to the inside thereof. Therefore, if the slider 41 is operated to be pushed and pulled, the needle tip 35b of the leading end of the puncture pipe body 35 moves in and out from the leading end of the sheath member 34.

Further, the guide hole 42 having a predetermined length is provided in the axial direction in the peripheral body portion of the casing 40 and the pin 43 which is inserted into the guide hole 42 is attached to the slider 41. Both ends of the guide hole 42 are bent at about 90°, thereby becoming positioning hole portions 42a and 42b directed in the circumferential direction. A movement stroke of the slider 41 is regulated by the entire length of the guide hole 42 and the stroke end positions of both ends are regulated.

A state where the slider 41 is drawn out of the casing 40 and thus the puncture pipe body 35 is drawn into the sheath member 34 is the retreated position of the puncture pipe body 35, and if the pin 43 is shifted from the guide hole 42 to the positioning hole portion 42a, the puncture pipe body 35 can be stably retained at the retreated position. At the retreated position, the needle tip 35b of the puncture pipe body 35 is located slightly inside the leading end of the sheath member 34, and thus a state where the needle tip 35b is completely covered by the sheath member 34 is created, whereby at the time of insertion into the treatment tool insertion channel 21, or the like, the needle tip 35b does not stab another object or is not caught on another object, and thus safety is secured and an insertion operation into the treatment tool insertion channel 21 can also be smoothly performed.

On the other hand, if the slider 41 is pushed into the casing 40, the puncture pipe body 35 is led out of the sheath member 34. Then, if the pin 43 is arranged at a position where the pin 43 is engaged with the positioning hole portion 42b of the leading end of the guide hole 42, the puncture pipe body 35 protrudes by a predetermined length from the sheath member 34. This is the working position where puncture can be performed, and a protruding length at this working position becomes the maximum stab length into the body.

Here, the maximum stab length of the puncture pipe body 35 is a length in which the puncture pipe body 35 is stabbed to a position where the sheath member 34 comes into contact with the body cavity inner wall and, also in this state, there is a need to make the needle tip 35b enter the ultrasonic observation visual field by the ultrasonic transducer unit 16. Therefore, the maximum stab length of the puncture pipe body 35 is regulated to the ultrasonic observation visual field.

In order to shift the pin 43 which is provided on the slider 41 side, from the guide hole 42 of the casing 40 to the positioning hole portions 42a and 42b at the above-described retreated position and the working position and then stabilize the pin 43 at these positions, it is only necessary to relatively turn the casing 40 and the slider 41. In addition, if the widths of shift portions from the guide hole 42 to the positioning hole portions 42a and 42b are made slightly narrower than the outer diameter dimension of the pin 43, a click feeling can be obtained at the time of the shift and also the pin 43 can be stably retained.

As described above, in an operation of only the first operating means 37, the positional relationship between the cutter pipe body 36 and the puncture pipe body 35 does not change. Therefore, if the second operating means 38 (the operating rod 44) is operated, the cutter pipe body 36 reciprocates by a predetermined stroke in a state where it comes into close contact with the inside of the puncture pipe body 35. A movement range of the cutter pipe body 36 is between the drawn-in position (a position shown by a solid line in Fig. 8) where the blade edge 36a is located on the base end side of the suction hole 35c and the pushed-out position (a position shown by an imaginary line in Fig. 8) where the blade edge 36a completely passes the suction hole 35c, but does not protrude from the opened leading end of the puncture pipe body 35.

The second operating means 38 for displacing the cutter pipe body 36 back and forth between the two positions in this manner is constituted by providing a guide tube portion 41a making the base end side of the slider 41 in the first operating means 37 have a larger diameter of a predetermined length, and inserting a slide portion 44a on the leading end side of the operating rod 44, to which the base end portion of the cutter pipe body 36 is connected, into the guide tube portion 41a so as to be able to slide back and forth by a predetermined stroke. Then, a guide hole 45 is formed in a peripheral body portion of the guide tube portion 41a, both ends of the guide hole 45 are bent at about 90°, thereby becoming positioning hole portions 45a and 45b, and a pin 46 which is guided by the guide hole 45 is attached to the slide portion 44a of the operating rod 44.

Therefore, by pushing and pulling the operating rod 44 with respect to the guide tube portion 41a of the slider 41, the blade edge 36a of the leading end of the cutter pipe body 36 moves back and forth to pass the suction hole 35c formed in the main body pipe 35a of the puncture pipe body 35.

If the pin 46 is engaged with the positioning hole portion 45a in the guide hole 45, the blade edge 36a of the cutter pipe body 36 is located at the drawn-in position further on the base end side than the suction hole 35c of the puncture pipe body 35. On the other hand, if the slider 41 is pushed and the pin 46 is then engaged with the positioning hole portion 45b, the blade edge 36a of the cutter pipe body 36 passes the suction hole 35c, thereby being displaced to the pushed-out position.

Here, at the pushed-out position, the blade edge 36a is located at a position where it does not protrude from the main body pipe 35a of the puncture pipe body 35. Then, the cutter pipe body 36 is stably retained at each of the drawn-in position and the pushed-out position by engaging the pin 46 with the positioning hole portion 45a or 45b. A shift of the pin 46 from the guide hole 45 to the positioning hole portion 45a or 45b can be performed by relatively turning the slider 41 and the operating rod 44, and if the width of the shift portion is made slightly narrower than the diameter of the pin 46, a click feeling can be obtained and stability of the pin 46 can be secured.

The cutter pipe body 36 also functions as a fluid pathway. This fluid pathway is constituted by as a suction pathway for making negative pressure act on the suction hole 35c and a pathway in which liquid such as formalin solution is pumped so as to discharge a tissue housed in the cutter pipe body 36. Then, as an extending portion of the fluid pathway in the cutter pipe body 36, the flow path 47 is formed in the operating rod 44. Then, the base end portion of the operating rod 44 becomes the luer lock portion 44b to which the syringe 33 for suction or for liquid pumping is detachably connected.

In addition, the needle-stick prevention mechanism for reliably preventing a worker from being stabbed with the needle tip 35b of the puncture pipe body 35 when disposing the tissue harvesting apparatus 30 after use is provided at the operating rod 44 of the operating unit 32. The details of the needle-stick prevention mechanism will be described later.

This embodiment is constituted as described above, and next, a method of harvesting a tissue in the body by using the tissue harvesting apparatus 30 will be described.

First, the leading end portion main body 12 of the ultrasonic endoscope is arranged at a predetermined position with respect to the body cavity inner wall S. In this state, if the tissue harvesting place T in the body is captured in the observation visual field of the ultrasonic transducer unit 16 constituting the ultrasonic observation section 14, the catheter 31 is inserted into the treatment tool insertion channel 21 such that the leading end portion thereof is located in the vicinity of the leading end of the treatment tool lead-out portion 18. Further, the syringe 33 remains connected to the luer lock portion 44b constituting the operating rod 44. However, as the syringe 33, a syringe for suction is used.

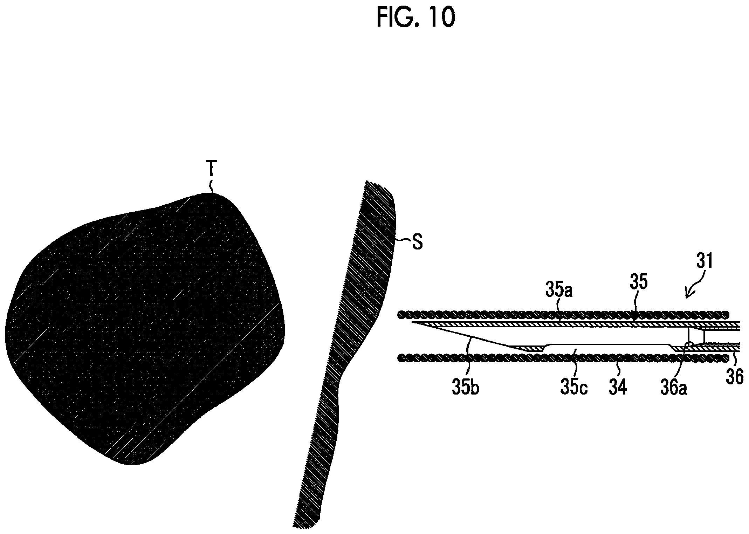

Here, as shown in Fig. 10, the leading end portion of the puncture pipe body 35 in the catheter 31 is covered by the sheath member 34, and in this state, the slider 41 in the first operating means 37 is operated so as to be pushed into the casing 40, whereby the puncture pipe body 35 is led out of the sheath member 34. In this way, the needle tip 35b of the leading end of the puncture pipe body 35 is stuck into the body from the body cavity inner wall S.

Since a sticking route of the puncture pipe body 35 into the body can be captured in the ultrasonic observation visual field, the sticking operation can be safely performed and it is possible to reliably target the tissue harvesting place T.

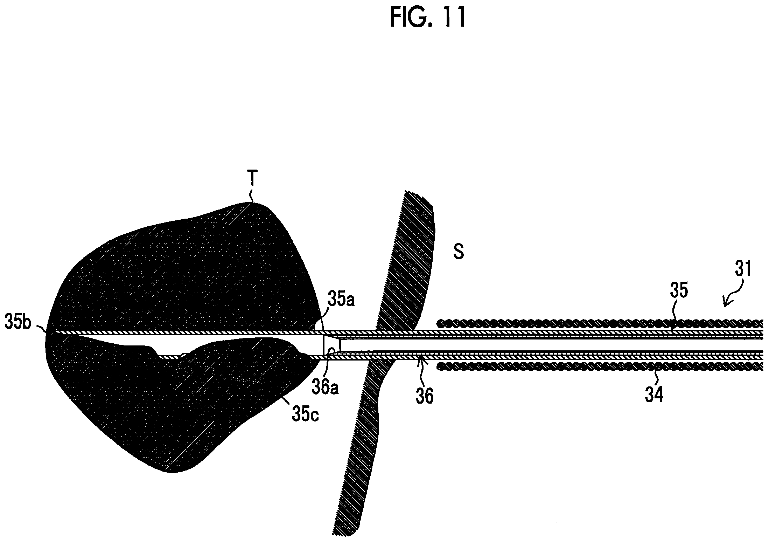

If the needle tip 35b of the puncture pipe body 35 advances and the suction hole 35c faces the living body tissue harvesting place T, the syringe is operated, thereby making the inside of the cutter pipe body 36 be under negative pressure. Due to the action of the negative pressure, a tissue enters the suction hole 35c, as shown in Fig. 11. In addition, since the leading end of the puncture pipe body 35 is also opened and the negative pressure also acts on the leading end opening portion, the living body tissue also enters the puncture pipe body 35 through the leading end opening portion. However, since the opening portions of the two places are separated from each other by a wall portion of the main body pipe 35a of the puncture pipe body 35, the tissues suctioned from the two places are in a state of being separated from each other. In addition, since the needle tip 35b is cut obliquely forward from the side where the suction hole 35c is formed, the living body tissue is pushed away so as to follow the oblique wall as the needle tip 35b is stuck into the body. As a result, if a compression force in a direction shown by an arrow in Fig. 11 acts on the living body tissue and negative pressure acts on the cutter pipe body 36, the tissue smoothly enters the puncture pipe body 35 through the suction hole 35c opened to the side. Thus, since almost the entirety of the inner diameter of the puncture pipe body 35 functions as an housing portion for the suctioned living body tissue, a wide housing space can be obtained, and thus a sufficient amount of tissue can be taken in.

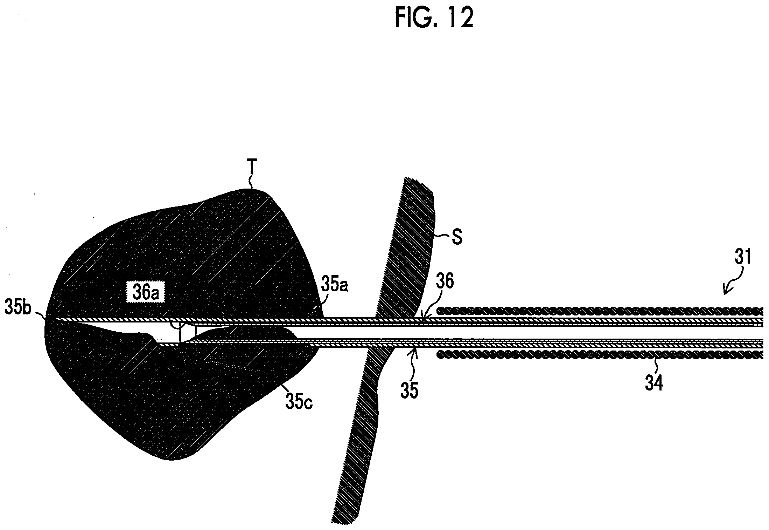

If a given amount of living body tissue enters the puncture pipe body 35 through the suction hole 35c, by pushing the slide portion 44a of the operating rod 44 into the slider 41, as shown in Fig. 12, the blade edge 36a of the cutter pipe body 36 advances, thereby cutting the living body tissue entering the puncture pipe body 35 and taking it in the cutter pipe body 36. At this time, negative pressure remains acting on the inside of the cutter pipe body 36. However, further suction power is prevented from acting. That is, the syringe is maintained so as not to move. In this way, the living body tissue is harvested from the body and retained in the cutter pipe body 36. In addition, since the blade edge 36a of the cutter pipe body 36 does not act on the living body tissue suctioned through the leading end opening of the puncture pipe body 35, the living body tissue is not cut, and thus damage to the living body tissue can be minimized.

After the living body tissue is harvested by the catheter 31 of the tissue harvesting apparatus 30 in this way, the catheter 31 is extracted from the treatment tool insertion channel 21. At this time, the cutter pipe body 36 is maintained at the pushed-out position with respect to the puncture pipe body 35. However, the catheter 31 can be safely pulled out by displacing the puncture pipe body 35 to the retreated position with respect to the sheath member 34. Then, instead of the syringe for suction, a syringe that pumps, for example, formalin solution is connected to the luer lock portion 44b, and the harvested tissue can be transferred to a test tube B or the like by pumping the formalin solution from the syringe into the cutter pipe body 36.

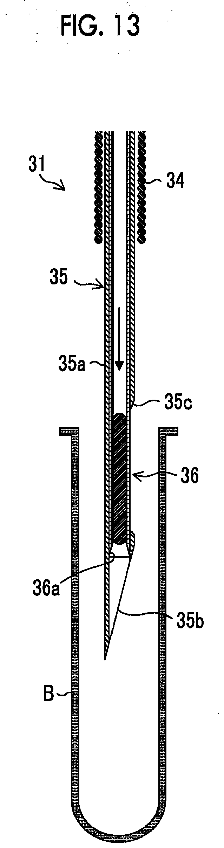

Here, as shown in Fig. 13, since the leading end of the puncture pipe body 35 is opened and the suction hole 35c is covered by the cutter pipe body 36, the harvested tissue is reliably discharged from the leading end of the puncture pipe body 35 by the pressure of the formalin solution or the like, as shown by an arrow in the same drawing, and thus a concern such as the tissue suffering damage at the time of the transfer work does not occur and a sample in very good condition is taken.

In addition, although the puncture pipe body 35 has the suction hole 35c formed in the side surface thereof, if the cutter pipe body 36 is displaced to the pushed-out position, the suction hole 35c can be closed substantially. Further, the syringe 33 is detachably connected to the operating rod 44 in the base end portion of the cutter pipe body 36. From the above, a drug solution can be injected from the syringe 33 in a state where the suction hole 35c is closed, and if a syringe for suction is connected, suction of a body fluid or the like also becomes possible. Therefore, the tissue harvesting apparatus 30 can also be used as a puncture treatment tool for performing suction, drug solution injection, or the like.

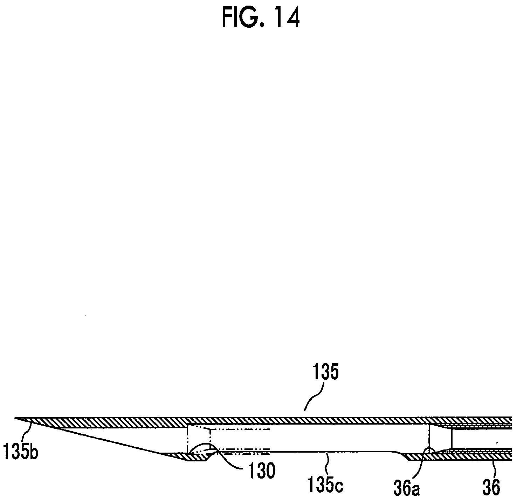

Further, as shown in Fig. 14, a step 130 can also be formed between a needle tip 135b and a suction hole 135c in a puncture pipe body 135. The diameter of a leading end opening of the puncture pipe body 135 which is formed by the step 130 is made equal to or larger than the inner diameter dimension of the cutter pipe body 36 which is housed in the inside, thereby avoiding a site of the step becoming a constricting portion. Further, it is preferable that a formation position of the step 130 be set to be a position where the blade edge 36a does not come into contact with the step 130 when the cutter pipe body 36 is displaced to a pushed-in position in the normal state. According to such a configuration, as shown by an imaginary line in the same drawing, when the blade edge 36a constituting the leading end portion of the cutter pipe body 36 is displaced to the pushed-out position, the blade edge 36a can be completely prevented from unnecessarily protruding from the puncture pipe body 135.

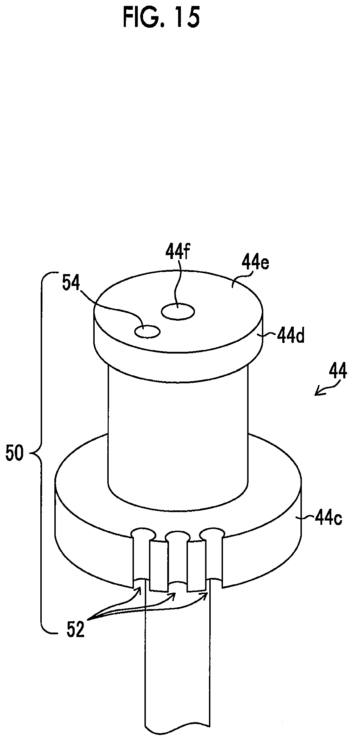

Next, the needle-stick prevention mechanism which is provided at the operating unit 32 of the tissue harvesting apparatus 30 will be described. The needle-stick prevention mechanism is for reliably preventing a worker from being stabbed with the needle tip 35b of the puncture pipe body 35 when disposing the tissue harvesting apparatus 30 as in the first embodiment or the second embodiment which has finished harvesting of a living body tissue by doing as described above. The operating rod 44 of the operating unit 32 in the tissue harvesting apparatus 30 of the first embodiment or the second embodiment shown in Fig. 2 or 7 is shown in Fig. 15 in an enlarged manner. As shown in the same drawing, a needle-stick prevention mechanism 50 includes a plurality of (in an example shown in the same drawing, three) locking grooves 52, 52, and 52 provided to extend at an outer circumferential portion of a first flange portion 44c on the leading end side of the operating rod 44 which is arranged at the rearmost end of the operating unit 32, and a needle housing hole 54 formed in an end face 44e of a second flange portion 44d of the base end portion of the operating rod 44. In addition, an opening 44f provided in the end face 44e is an opening into which a syringe is inserted.

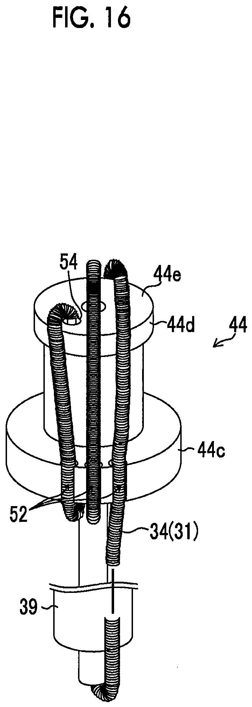

The details of the configurations of the locking groove 52 and the needle housing hole 54 will be described later and a form when disposing the tissue harvesting apparatus 30 after use is shown in Fig. 16. When disposing the tissue harvesting apparatus 30, as shown in Fig. 16, the sheath member 34 (and the catheter 31 constituted by the puncture pipe body 35 inserted and arranged in the inside of the sheath member 34, and the cutter pipe body 36 in the second embodiment) extending toward the front from the connecting member 39 (refer to Figs. 2, 4, 7, and 9) of the leading end of the operating unit 32 is curved toward the rear (the base end side) in the vicinity of the base end portion. Then, the sheath member 34 curved in the vicinity of the base end portion is fitted into any one of the locking grooves 52, 52, and 52 formed in a plurality, whereby the sheath member 34 is locked by the locking groove 52. Then, an extra length portion of the sheath member 34, which becomes positioned further toward the leading end side than the portion locked by the locking groove 52, is wound in the form of a loop (the form of a loop in which a direction perpendicular to the axial direction of the operating rod 44 is set to be a direction perpendicular to a winding direction) winding around the front side of the first flange portion 44c of the operating rod 44 and the rear side of the second flange portion 44d, as in the same drawing, and also fitted into and locked by any one of the non-fitted locking grooves 52 when passing through the formation places of the locking grooves 52, 52, and 52 of the first flange portion 44c.

If a state where the sheath member 34 which is wound in the form of a loop and fitted into all of the locking grooves 52, 52, and 52 is created in this way, the extra length portion of the sheath member 34 is shortened. Then, the needle tip 35b of the puncture pipe body 35 is exposed from the leading end of the sheath member 34 and inserted into the needle housing hole 54 formed in the end face 44e of the second flange portion 44d, whereby the needle tip 35b of the puncture pipe body 35 and a portion adjacent thereto are housed in the inside of the needle housing hole 54. In this way, during subsequent disposal work, winding in the form of a loop is not easily released, and thus a contingency situation such as a worker being stabbed with the needle tip 35b of the puncture pipe body 35 is reliably prevented.

In addition, in a case where the sheath member 34 is wound in the form of a loop, the sheath member 34 may also be wound in the form of a loop after the needle tip 35b of the puncture pipe body 35 is first housed in the inside of the needle housing hole 54. Further, the winding direction of the sheath member 34 may also be a direction (a circumferential direction) around the axis of the operating rod 44, rather than a winding direction such as the sheath member 34 reciprocating in the axial direction of the operating rod 44, as in Fig. 16, and in this case, it is desirable if the locking groove 52 is formed in the circumferential direction.

Subsequently, the details of the locking groove 52 and the needle housing hole 54 of the needle-stick prevention mechanism 50 described above will be described.

The locking groove 52 is provided in parallel at a plurality of places of the operating rod 44 in the outer circumferential portion of the first flange portion 44c of the operating rod 44, as described above. The number thereof may also be the number corresponding to the number of turns of the sheath member 34 in a case where the sheath member 34 is wound in the form of a loop, as in Fig. 16, and a configuration is also acceptable in which the number is set to be about three, as in Fig. 16, regardless of the number of turns, and the sheath member 34 is fitted each time it is wound multiple times.

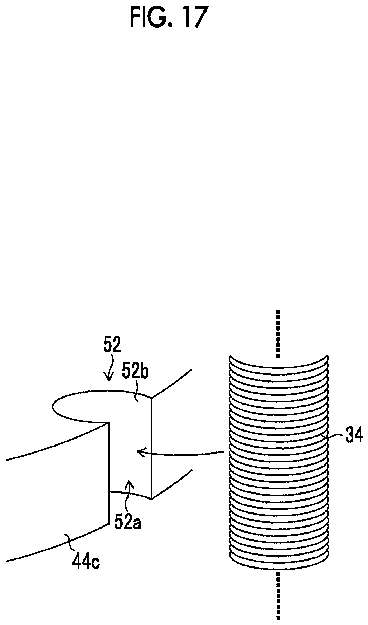

Each locking groove 52 is formed so as to have an opening 52a extending in parallel to the axial direction of the operating rod 44 and an inner wall surface 52b, as in, for example, Fig. 17, and the inner wall surface 52b is formed by a curved surface that becomes an arc having a diameter approximately conforming to the outer diameter of the sheath member 34 and having a central angle larger than 180 degrees, in a cross-section perpendicular to the axial direction. According to this, the width in the circumferential direction (the width in a short direction) of the opening 52a becomes smaller than the outer diameter of the sheath member 34.

According to this, if the sheath member 34 is pushed toward the inside while making it follow the opening 52a of the locking groove 52, the sheath member 34 or the opening 52a is deformed, and thus the sheath member 34 enters the inside of the locking groove 52, whereby the sheath member 34 is locked in a state where the entirety of the inner wall surface 52b of the locking groove 52 and the outer circumferential surface of the sheath member 34 come into close contact with each other. Then, since the width of the opening 52a is smaller than the outer diameter of the sheath member 34, the sheath member 34 fitted into the inside of the locking groove 52 once does not easily come off.

In addition, the form of the locking groove 52 is not limited thereto and may also have a form of locking the sheath member 34 by simply press-fitting it between the inner wall surfaces facing each other of a rectangular groove, and other forms are also acceptable.

The needle housing hole 54 is drilled to a depth of, for example, about 1 cm in the end face 44e of the second flange portion 44d of the operating rod 44, as described above. The needle housing hole 54 may also be a hole that is simply fitted to the puncture pipe body 35. That is, a form is possible in which the needle housing hole 54 is set to be a hole having approximately the same diameter as the outer diameter of the puncture pipe body 35, whereby, if the puncture pipe body 35 is inserted, the inner circumferential surface of the needle housing hole 54 comes into close contact with the outer circumferential surface of the puncture pipe body 35 such that the puncture pipe body 35 does not easily come out of the needle housing hole 54 due to a frictional force.

On the other hand, as another form, a form may also be adopted in which the needle housing hole 54 is set to be a hole having an arbitrary shape larger than the outer diameter of the puncture pipe body 35 (a hole having an arbitrary shape such as a circle, a quadrilateral, or the like in a cross-section perpendicular to an insertion direction of the puncture pipe body 35 and having a size in which the puncture pipe body 35 can be inserted) and come off prevention means (a come off prevention member) which fixedly supports the puncture pipe body 35 so as not to easily come out of the needle housing hole 54 is provided in the inside or the like of the needle housing hole 54.

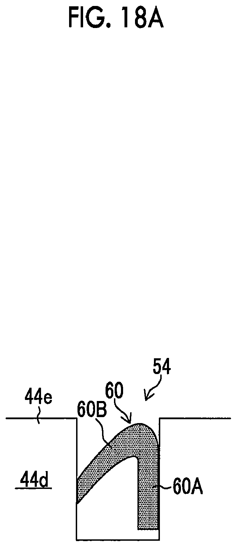

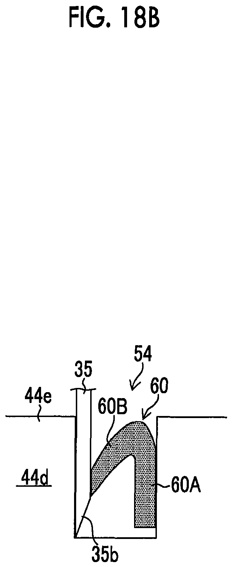

A first embodiment of the needle housing hole 54 provided with the come off prevention means is shown in Figs. 18A and 18B. Figs. 18A and 18B are cross-sectional views in which the needle housing hole 54 is cut in a plane in the insertion direction of the puncture pipe body 35 (a depth direction). As shown in Fig. 18A, as the come off prevention means, a stopper 60 (a pressing member) for pressing the puncture pipe body 35 to the side wall surface of the needle housing hole 54 is provided in the inside of the needle housing hole 54. The needle housing hole 54 is formed as a hole of, for example, a rectangular parallelepiped shape, and the stopper 60 is formed so as to have a base end portion 60A which is arranged along one surface of four side wall surfaces of the rectangular parallelepiped-shaped hole and also fixedly attached to the side wall surface by an adhesive or the like, and an extending portion 60B which is provided to extend from an upper end (on the opening side of the needle housing hole 54) of the base end portion 60A and extends to a position where the base end portion 60A comes into contact with the side wall surface that faces the side wall surface to which the base end portion 60A is fixedly attached.

The stopper 60 constituted by the base end portion 60A and the extending portion 60B is integrally formed by an elastic member such as a rubber member and arranged in the inside of the needle housing hole 54 in a state where a leading end portion of the extending portion 60B comes into contact with the side wall surface of the needle housing hole 54, whereby the stopper 60 is elastically deformed in a direction (the counterclockwise direction in the drawing) in which the amount of bending of a bent portion that becomes a connecting portion of the base end portion 60A and the extending portion 60B becomes larger than the amount of bending when an external force is removed. Therefore, the extending portion 60B is in a state of having a biasing force such that the leading end portion is displaced to the opening side with the bent portion as a fulcrum.

According to the needle housing hole 54 of the first embodiment, if the puncture pipe body 35 is inserted into the needle housing hole 54 from the needle tip 35b, as shown in Fig. 18B, the puncture pipe body 35 deforms the stopper 60 in a direction to increase the amount of bending of the bent portion of the stopper 60, thereby creating a gap between the extending portion 60B and the side wall surface of the needle housing hole 54, and passes through the gap, thereby reaching a position where the puncture pipe body 35 comes into contact with the bottom of the needle housing hole 54. In this way, the puncture pipe body 35 is pressed to the side wall surface of the needle housing hole 54 by a biasing force or the like from the bent portion acting on the extending portion 60B, thereby being fixedly supported in a state where the puncture pipe body 35 does not easily come out of the needle housing hole 54. Further, dimple working (working to form a large number of small depressions) is carried out on the outer circumferential surface of at least the leading end portion of the puncture pipe body 35 such that the outer circumferential surface is easily reflected in an ultrasonic image, and since a large frictional force acts due to contact between the outer circumferential surface of the puncture pipe body 35 subjected to the dimple working and the leading end portion of the extending portion 60B of the stopper 60, the puncture pipe body 35 is fixedly supported in a state where it does not more easily come out of the needle housing hole 54.

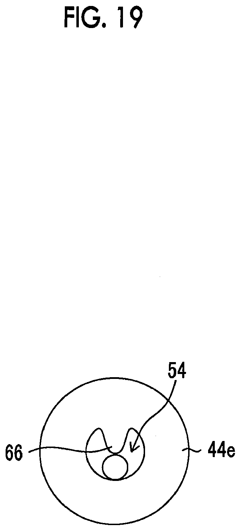

A second embodiment of the needle housing hole 54 provided with the come off prevention means is shown in Fig. 19. Fig. 19 is a plan view showing the needle housing hole 54 from the insertion direction of the puncture pipe body 35. As shown in the same drawing, as the come off prevention means, a convex portion 66 (a convex member) for holding the puncture pipe body 35 between the convex portion 66 and the side wall surface of the needle housing hole 54 is provided in the inside of the needle housing hole 54. The needle housing hole 54 has a columnar shape larger than the outer diameter of the puncture pipe body 35, as a basic shape, and the convex portion 66 is arranged so as to protrude toward the center of the hole from a portion of the side wall surface of the hole. Further, a leading end portion of the convex portion 66 is formed in a round shape.

Here, the convex portion 66 may also be a portion formed to protrude from the side wall surface of the needle housing hole 54 (a member formed integrally with the second flange portion 44d) and may also be an independent member fixedly attached to the side wall surface of the needle housing hole 54 by an adhesive or the like. In either case, the shape of the needle housing hole 54 is equivalent to a shape in which the shape of the convex portion 66 is harvested from a columnar shape. Further, in the latter case, it is preferable to form a member of the convex portion 66 by an elastic member having elasticity comparable to plastic. In addition, the convex portion 66 may also be a portion provided to extend from the position of the opening of the needle housing hole 54 to the position of the bottom and may also be a portion which is provided in a partial range from the position of the opening to the position of the bottom.

According to the needle housing hole 54 of the second embodiment, in a case where the puncture pipe body 35 having a predetermined diameter is inserted into the needle housing hole 54, a state where the puncture pipe body 35 is held between the wall surface of the convex portion 66 and the other side wall surface of the needle housing hole 54 is created. However, the maximum diameter of the puncture pipe body 35 capable of being inserted varies according to an insertion position, and for example, in a case where a position at which the outer circumferential surface of the puncture pipe body 35 comes into contact with the wall surface of the convex portion 66 is continuously changed, the maximum diameter of the puncture pipe body 35 capable of being inserted at each position also changes continuously.

Therefore, the needle housing hole 54 is formed such that the maximum diameter of a puncture pipe body capable of being inserted approximately conforms to at least the outer diameter of the puncture pipe body 35 which is actually used, or becomes larger than the outer diameter of the puncture pipe body 35. In particular, in a case where a problem in which it becomes difficult for the puncture pipe body 35 to be stuck into the needle housing hole 54 due to manufacturing error in the needle housing hole 54 or the puncture pipe body 35, a change in temperature, or the like can be reliably avoided, the needle housing hole 54 is formed such that the maximum diameter of a puncture pipe body capable of being inserted into the needle housing hole 54 is larger than the outer diameter of the puncture pipe body 35.

When inserting the puncture pipe body 35 into the needle housing hole 54, a position where the puncture pipe body 35 can be easily inserted into the needle housing hole 54 is searched for and at the position, the puncture pipe body 35 is inserted to a position where the puncture pipe body 35 comes into contact with, for example, the bottom of the needle housing hole 54. Then, the puncture pipe body 35 is moved in a direction perpendicular to the insertion direction, thereby being moved to a position where the puncture pipe body 35 is solidly held between the wall surface of the convex portion 66 and the other side wall surface of the needle housing hole 54. In this way, the puncture pipe body 35 is fixedly supported in a state where it does not easily come out of the needle housing hole 54.

In addition, the convex portion 66 may also be a plate-like member which is arranged in only the vicinity of the opening, for example. In this case, since the convex portion 66 is elastically deformed in the depth direction when inserting the puncture pipe body 35, thereby having a biasing force to press the puncture pipe body 35 to the side wall surface of the needle housing hole 54 after the insertion of the puncture pipe body 35, and since a frictional force acts between the convex portion 66 and the outer circumferential surface of the puncture pipe body 35 subjected to the dimple working, the puncture pipe body 35 is fixedly supported so as not to easily come out of the needle housing hole 54.

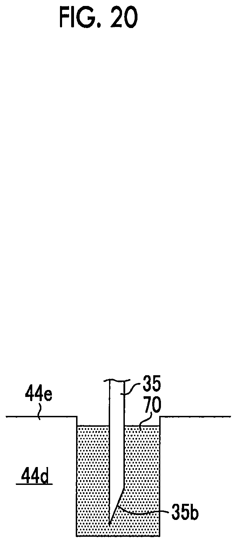

A third embodiment of the needle housing hole 54 provided with the come off prevention means is shown in Fig. 20. Fig. 20 is a cross-sectional view in which the needle housing hole 54 is cut in a plane in the insertion direction of the puncture pipe body 35 (a depth direction). As shown in the same drawing, as the come off prevention means, a rubber member 70 (an elastic member) in which a hole housing the needle tip 35b of the puncture pipe body 35 is drilled by the needle tip 35b when housing the needle tip 35b of the puncture pipe body 35 in the needle housing hole 54 is provided in the inside of the needle housing hole 54.

The needle housing hole 54 is formed as a hole having an arbitrary shape such as a columnar shape or a rectangular parallelepiped shape, for example, and the rubber member 70 is formed in a shape approximately matching to the shape of the needle housing hole 54 and fitted so as to fill up the entire inside of the needle housing hole 54 almost without a gap. In addition, the rubber member 70 may also fill up an approximately entire range from the position of the opening of the needle housing hole 54 to the position of the bottom and may also fill up a partial range from the position of the opening to the position of the bottom.

According to the needle housing hole 54 of the third embodiment, when the puncture pipe body 35 is inserted into the needle housing hole 54, the needle tip 35b of the puncture pipe body 35 is stuck at an arbitrary position of the rubber member 70 in the needle housing hole 54 and pushed to a position where it comes into contact with the bottom of the needle housing hole 54 (or to an arbitrary depth) as it is, while forming a hole in the rubber member 70 by the needle tip 35b of the puncture pipe body 35. In this way, the puncture pipe body 35 is fixedly supported in a state where it does not more easily come out of the needle housing hole 54, by the elastic force of the rubber member 70 and a frictional force with the rubber member 70. In addition, as an alternative to the rubber member 70, an elastic member in which a hole can be formed by the puncture pipe body 35 can be used.

In the needle-stick prevention mechanism 50 described above, the locking groove 52 which is provided in the first flange portion 44c of the operating rod 44 may also be provided in the same way on the opposite side across the center of the first flange portion 44c so as to lock the sheath member 34 wound in the form of a loop, on both sides of the first flange portion 44c. Further, a form in which the locking groove 52 is not provided at all is also acceptable. However, in the case of a form in which the locking groove 52 is not provided, compared to a case where the locking groove 52 is provided, since the winding in the form of a loop of the sheath member 34 is prone to be disturbed, it is good if after the sheath member 34 is wound in the form of a loop, the sheath members 34 are bundled by a string, an adhesive tape, or the like, or fixedly attached to a predetermined site such as the first flange portion 44c of the operating rod 44 by an adhesive tape, or the like. Even in a case where the locking groove 52 is provided, a situation in which the winding is released can be reliably prevented by performing such a measure after the sheath member 34 is wound in the form of a loop.

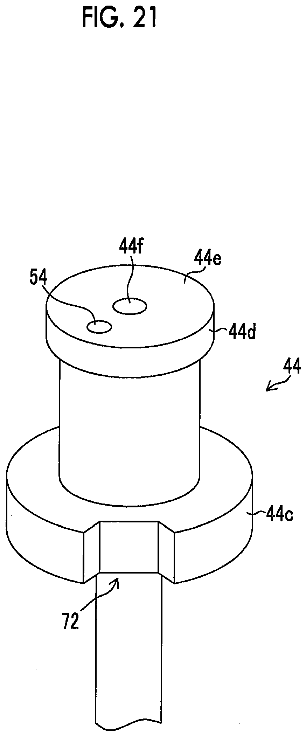

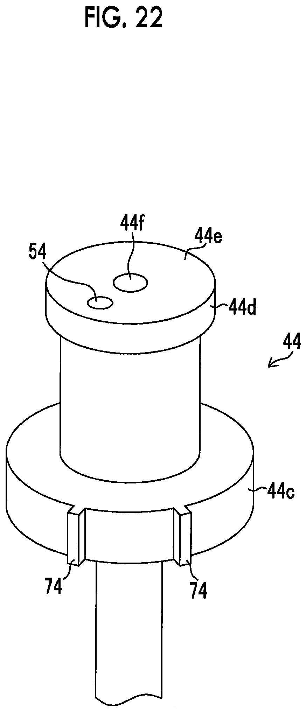

Further, the locking groove 52 is for preventing the release of the winding due to a movement in a direction perpendicular to the winding direction of a loop of the sheath member 34 wound so as to surround the first flange portion 44c and the second flange portion 44d. However, as in Fig. 21, a configuration is also acceptable in which a concave portion 72 is formed in an outer circumferential portion of the first flange portion 44c and the sheath member 34 wound in the form of a loop is housed in the concave portion 72, thereby preventing the release of the winding. Further, as in Fig. 22, a configuration is also acceptable in which two convex portions 74 and 74 are formed to protrude at an outer circumferential portion of the first flange portion 44c and the sheath member 34 wound in the form of a loop is housed between the two convex portions 74 and 74, thereby preventing the release of the winding. The concave portion 72 and the convex portions 74 and 74 may also be formed in the same way on the opposite side that becomes a symmetrical position with respect to the center of the first flange portion 44c.

Further, the locking groove 52 or the needle housing hole 54 of the needle-stick prevention mechanism 50 described above may also be provided at any position of the operating unit 32. For example, the locking groove 52 may also be provided at the second flange portion 44d and the needle housing hole 54 may also be provided at the first flange portion 44c. Further, in a case where the locking groove 52 or the needle housing hole 54 is provided at the operating rod which is arranged at the rearmost end of the operating unit 32, as in the above-described embodiments, types of operation being performed by the operating rod are not limited to the cases of the embodiments described above. The tissue harvesting apparatus 30 in which the needle-stick prevention mechanism 50 is provided is also not limited to the tissue harvesting apparatuses 30 of the first and second embodiments shown in Figs. 2, 7, and the like.

Further, the needle-stick prevention mechanism 50 described above may also be provided with a mounting member which is detachably mounted with respect to the operating unit 32, rather than being directly provided in the operating unit 32. For example, in the tissue harvesting apparatuses 30 of the embodiments described above, the locking groove 52 and the needle housing hole 54 may also be formed in a cylindrical mounting member having a concave portion at an end face (a bottom surface) thereof, which is mounted by being fitted to the second flange portion 44d of the operating rod 44 which is arranged at the rearmost end of the operating unit 32, from the rear end side. Also in this case, the locking groove 52 need not be necessarily provided. In particular, such a form is effective in a case where a space to form a needle-stick prevention mechanism is not present in a tissue harvesting apparatus having a small operating unit, and it is good if a mounting member provided with the needle-stick prevention mechanism 50 described above is detachably mounted on the operating unit from the rear end side.

Next, a form of the mounting member (a needle-stick prevention tool) provided with the needle-stick prevention mechanism which is detachably mounted on the operating unit of the tissue harvesting apparatus, as described above, will be described. In addition, in the following description and the drawings, the locking groove 52 and the needle housing hole 54 which are configured in the same way as those in the embodiments described above are denoted by the same reference numerals and the detailed description thereof is omitted. Further, various forms (the forms shown in Figs. 21, 22, and the like, or the form of the come off prevention means) described above in connection with the locking groove 52 and the needle housing hole 54 can also be similarly applied to a needle-stick prevention tool which is described below.

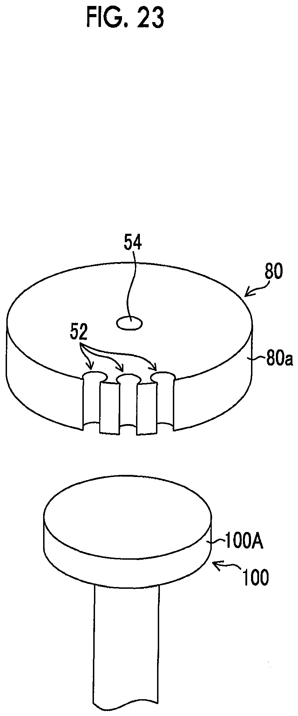

Fig. 23 is a perspective view showing a first embodiment of the needle-stick prevention tool. In the same drawing, reference numeral 100 denotes an operating rod which is arranged at a rearmost end in an operation unit of an arbitrary tissue harvesting apparatus. In the tissue harvesting apparatuses 30 of the first and second embodiments shown in Figs. 2, 7, and the like, the second flange portion 44d in the operating rod 44 of the operating unit 32 is equivalent thereto. Further, in tissue harvesting apparatuses of other forms, for example, an operating rod for a push-pull operation of an insertion member such as a puncture pipe body, a tube body (equivalent to the cutter pipe body 36 in the tissue harvesting apparatus 30 of the second embodiment described above) which is inserted into the puncture pipe body, or a stylet is provided. Such an operating rod is generally arranged at the rearmost end of the operating unit and the operating rod in this case is equivalent to the operating rod 100 in Fig. 23. However, there is no limitation thereto and the operating rod 100 in Fig. 23 represents an arbitrary operating rod which is arranged at the rearmost end of the operating unit.

The operating rod 100 in Fig. 23 is provided with, for example, a columnar flange portion 100a that an operator grips, and a needle-stick prevention tool 80 of this embodiment shown in the same drawing has a columnar main body 80a having an outer diameter larger than the flange portion 100a of the operating rod 100. The locking grooves 52, 52, and 52 are formed in an outer circumferential portion of the main body 80a and the needle housing hole 54 is formed in the upper surface of the main body 80a.

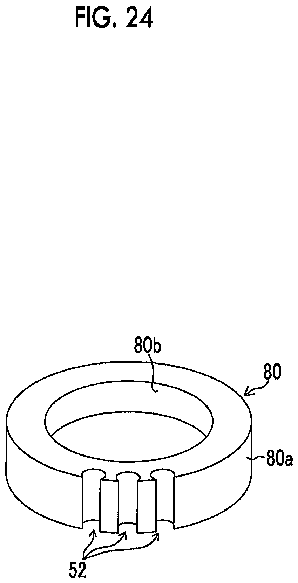

On the other hand, a columnar concave portion 80b is formed on the back surface side of the main body 80a, as shown in Fig. 24, and the inner diameter of the concave portion 80b is formed to a size approximately conforming to the outer diameter of the flange portion 100a of the operating rod 100.

Therefore, the flange portion 100a of the operating rod 100 can be fitted into and mounted in the concave portion 80b of the main body 80a of the needle-stick prevention tool 80 from the rear end side. In this way, the needle-stick prevention tool 80 can be detachably mounted in an operating unit of a tissue harvesting apparatus. Then, when the sheath member 34 is wound in the form of a loop around the needle-stick prevention tool 80, the sheath member 34 can be locked in the locking groove 52 and the puncture pipe body 35 can be inserted and fixedly supported in the needle housing hole 54.

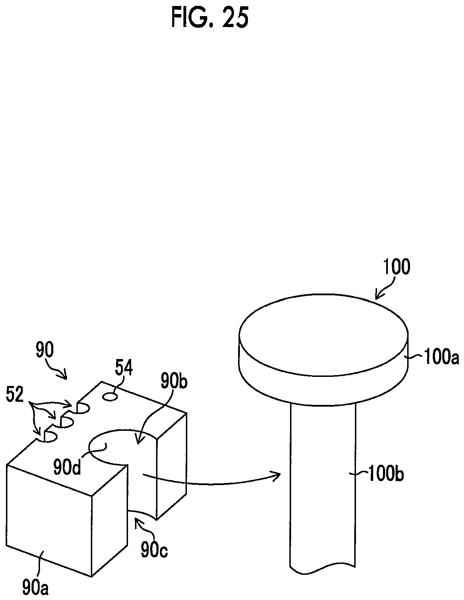

Fig. 25 is a perspective view showing a second embodiment of the needle-stick prevention tool. In the same drawing, the operating rod 100 represents an operating rod which is arranged at the rearmost end in an operating unit of an arbitrary tissue harvesting apparatus in the same way as in Fig. 23.

A needle-stick prevention tool 90 of this embodiment shown in the same drawing has a main body 90a having a rectangular parallelepiped shape, in which the locking grooves 52, 52, and 52 and the needle housing hole 54 are formed. A fitting groove 90b in the same direction as the locking groove 52 is formed in the side surface on the opposite side to the side surface of the main body 90a, in which the locking groove 52 is formed. The fitting groove 90b is formed so as to have an opening 90c extending in the same direction as the locking groove 52 and an inner wall surface 90d, and the inner wall surface 90d is formed by a curved surface that becomes an arc having a diameter approximately conforming to the outer diameter of a cylindrical portion 100b which is relatively long in the axial direction of the operating rod 100 and having a central angle larger than 180 degrees, in a cross-section perpendicular to a groove direction. According to this, the width in a short direction of the opening 90c becomes smaller than the outer diameter of the cylindrical portion 100b of the operating rod 100.

According to this, if the cylindrical portion 100b is arranged to follow the opening 90c of the fitting groove 90b of the needle-stick prevention tool 90 and then pushed into the opening 90c, the cylindrical portion 100b or the opening 90c is deformed, whereby the cylindrical portion 100b enters the inside of the fitting groove 90b and is fitted into the fitting groove 90b, and thus the needle-stick prevention tool 90 is mounted on the cylindrical portion 100b. Then, when the sheath member 34 is wound in the form of a loop around the needle-stick prevention tool 90 (an aspect in which the sheath member 34 is wound around only the needle-stick prevention tool 90 or an aspect in which the sheath member 34 is wound so as to surround the needle-stick prevention tool 90 and the flange portion 100a is possible), the sheath member 34 can be locked in the locking groove 52 and the puncture pipe body 35 can be inserted and fixedly supported in the needle housing hole 54. In addition, a form of the fitting groove 90b is not limited thereto and may also be a form to make the cylindrical portion 100b be mounted by simply press-fitting it between the inner wall surfaces facing each other of a rectangular groove, and other forms are also acceptable.

Further, the cylindrical portion 100b on which the needle-stick prevention tool 90 is mounted need not necessarily be a portion formed at an operating rod which is arranged at the rearmost end of an operating unit. In the tissue harvesting apparatus 30 of the first embodiment shown in Figs. 2, 4, and the like, the cylindrical portion 100b may also be equivalent to any portion of the operating unit 32, such as the casing 40 of the operating means 37, or the like, in addition to, for example, the slider 41 or the luer lock portion 44b of the operating rod 44, and in the tissue harvesting apparatus 30 of the second embodiment shown in Figs. 7, 9, and the like, the cylindrical portion 100b may also be equivalent to any portion of the operating unit 32, such as the casing 40 of the first operating means 37, the guide tube portion 41a of the second operating means, or the like, in addition to, for example, the slide portion 44a or the luer lock portion 44b of the operating rod 44.

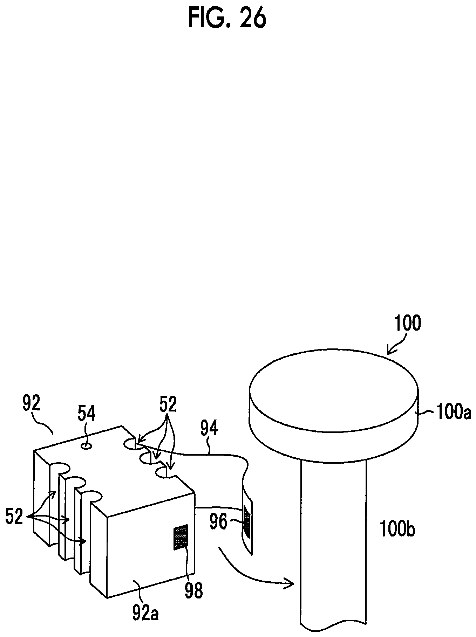

Fig. 26 is a perspective view showing a third embodiment of the needle-stick prevention tool which is detachably mounted on the cylindrical portion 100b of an operating unit of a tissue harvesting apparatus by a band using a hook-and-loop fastener, rather than being mounted on an operating unit by a fitting groove, as in the needle-stick prevention tool 90 in Fig. 25.

A needle-stick prevention tool 92 of this embodiment shown in the same drawing has a main body 92a having a rectangular parallelepiped shape, and in the main body 92a, the locking grooves 52, 52, 52, 52, 52, and 52 are formed in two surfaces which become the opposite sides to each other and the needle housing hole 54 is formed in the upper surface.

A base end of a band 94 is fixedly attached to the side surface of the main body 92a, where the locking groove 52 is not formed, and, for example, a hook-side member 96 of the hook-and-loop fastener is fixedly attached to a leading end of the band 94. On the other hand, for example, a loop-side member 98 of the hook-and-loop fastener is fixedly attached to the surface on the opposite side to the side surface to which the base end of the band 94 is fixedly attached, in the main body 92a.

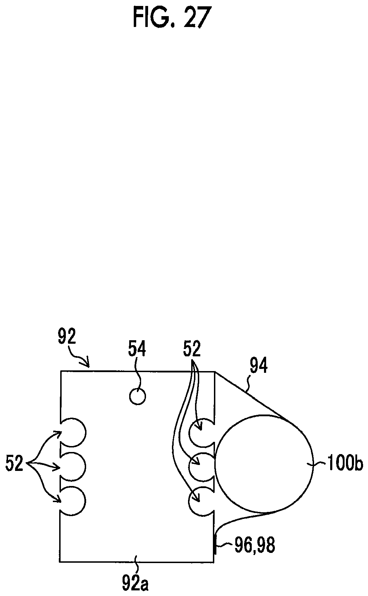

According to this, the needle-stick prevention tool 92 can be mounted on the cylindrical portion 100b by winding the band 94 around the cylindrical portion 100b of the operating rod 100 and then mounting the hook-side member 96 of the leading end of the band 94 on the loop-side member 98 of the main body 92a, as in Fig. 27. Then, when the sheath member 34 is wound in the form of a loop around the needle-stick prevention tool 92, the sheath member 34 can be locked in the locking groove 52 and the puncture pipe body 35 can be inserted and fixedly supported in the needle housing hole 54.

In addition, it is also possible to mount the needle-stick prevention tool 92 on the cylindrical portion 100b by doing as described above, after the sheath member 34 is wound in the form of a loop around the needle-stick prevention tool 92, and in this case, since the sheath member 34 wound in the form of a loop can also be housed along with the cylindrical portion 100b in a range surrounded by the band 94 and the side surface of the main body 92a, the release of the winding of the sheath member 34 can be more reliably prevented.

Further, as for a place on which the needle-stick prevention tool 92 is mounted, it is acceptable if it is a portion at which the needle-stick prevention tool 92 can be mounted by the band 94, and it is not limited to a specific portion.

## Claims

1. A tissue harvesting apparatus (30) comprising:
a needle tube (35) having a needle tip (35b) at a leading end thereof, the needle tip (35b) performing puncture;
an external tube (34) in which the needle tube (35) is inserted into inside and the needle tip (35b) of the needle tube (35) moves in and out from a leading end of the external tube; and
an operating unit (32) which is provided at a base end of the external tube and moves the needle tube in an axial direction; **characterized by**
a needle housing hole (54) which is arranged in the operating unit (32) and is configured to house and retain the needle tip (35b) of the needle tube (35) inside of the needle housing hole (54).

2. The tissue harvesting apparatus (30) according to claim 1, wherein a come off prevention member (60) fixedly supporting the needle tube (35) housed in the needle housing hole (54) is arranged in the inside of the needle housing hole (54).

3. The tissue harvesting apparatus (30) according to claim 2, wherein the come off prevention member (60) is a pressing member pressing the needle tube (35) housed in the needle housing hole (54) to a side wall surface of the needle housing hole (54).

4. The tissue harvesting apparatus (30) according to claim 2, wherein the come off prevention member (60) is a convex member holding the needle tube (35) housed in the needle housing hole (54) between the convex member and a side wall surface of the needle housing hole (54).

5. The tissue harvesting apparatus (30) according to claim 2, wherein the come off prevention member (60) is an elastic member in which a hole housing the needle tip (35b) is drilled by the needle tip (35b) when housing the needle tip (35b) of the needle tube (35) in the needle housing hole (54).

6. The tissue harvesting apparatus (30) according to any one of claims 1 to 5, wherein the operating unit (32) has a locking groove (52) locking the external tube (34) wound in the form of a loop along with the needle tube (35), when disposing the tissue harvesting apparatus (30).

7. The tissue harvesting apparatus (30) according to any one of claims 1 to 6, wherein the needle housing hole (54) is formed in an operating rod (44) which is arranged at a rearmost end of the operating unit (34).

8. The tissue harvesting apparatus (30) according to claim 7, further comprising: an insertion member (36) which is inserted into inside of the needle tube (35),
wherein the operating rod (44) is an operating rod moving the insertion member (36) in the axial direction with respect to the needle tube (35).

9. The tissue harvesting apparatus (30) according to claim 6, wherein the needle housing hole (54) and the locking groove (52) are formed in an operating rod (44) which is arranged at a rearmost end of the operating unit (32).

10. The tissue harvesting apparatus (30) according to claim 9, further comprising: an insertion member (36) which is inserted into inside of the needle tube (35),
wherein the operating rod (44) is an operating rod moving the insertion member (36) in the axial direction with respect to the needle tube (35).

11. The tissue harvesting apparatus (30) according to any one of claims 1 to 6, wherein the needle housing hole (54) is formed in a mounting member that is detachably mounted in the operating unit (32).

12. The tissue harvesting apparatus (30) according to claim 6, wherein the needle housing hole (54) and the locking groove (52) are formed in a mounting member (92) that is attachable and detachable in the operating unit (32).

## Patentansprüche

1. Gewebeentnahmevorrichtung (30), umfassend:
ein Nadelröhrchen (35) mit einer Nadelspitze (35b) an dessen vorderem Ende, wobei die Nadelspitze (35b) einen Einstich ausführt;
ein Außenröhrchen (34), in welchem das Nadelröhrchen (35) innen eingesetzt ist, wobei die Nadelspitze (35b) des Nadelröhrchens (35) sich in das und aus dem vorderen Ende des Außenröhrchens bewegt; und
eine Betätigungseinheit (32), die sich an einem Basisende des Außenröhrchens befindet und das Nadelröhrchen in axialer Richtung bewegt; **gekennzeichnet durch**
ein Nadelgehäuseloch (54), das in der Betätigungseinheit (32) angeordnet und konfiguriert ist zum Aufnehmen und Halten der Nadelspitze (35b) des Nadelröhrchens (35) im Inneren des Nadelgehäuselochs (54).

2. Gewebeentnahmevorrichtung (30) nach Anspruch 1, bei der im Inneren des Nadelgehäuselochs (54) ein Austritts-Sperrelement (60) angeordnet ist, welches das in dem Nadelgehäuseloch (54) untergebrachte Nadelröhrchen (35) fixiert hält.

3. Gewebeentnahmevorrichtung (30) nach Anspruch 2, bei der das Austritts-Sperrelement (60) ein Andrückelement ist, welches das in dem Nadelgehäuseloch (54) untergebrachte Nadelröhrchen (35) gegen eine Seitenwandfläche des Nadelgehäuselochs (54) drückt.

4. Gewebeentnahmevorrichtung (30) nach Anspruch 2, bei der das Austritts-Sperrelement (60) ein konvexes Teil ist, welches das in dem Nadelgehäuseloch (54) untergebrachte Nadelröhrchen (35) zwischen dem konvexen Teil und einer Seitenwandfläche des Nadelgehäuselochs (54) hält.

5. Gewebeentnahmevorrichtung (30) nach Anspruch 2, bei dem das Austritts-Sperrelement (60) ein elastisches Teil ist, in welchem ein die Nadelspitze (35b) aufnehmendes Loch von der Nadelspitze (35b) beim Aufnehmen der Nadelspitze (35b) des Nadelröhrchens (35) in dem Nadelgehäuseloch (54) aufnehmendes Loch gebohrt wird.

6. Gewebeentnahmevorrichtung (30) nach einem der Ansprüche 1 bis 5, bei der die Betätigungseinheit (32) eine Sperrnut (52) aufweist, die das in Form einer Schlaufe entlang dem Nadelröhrchen (35) gewundene Außenröhrchen beim Ablegen der Gewebeentnahmevorrichtung (30) verriegelt.

7. Gewebeentnahmevorrichtung (30) nach einem der Ansprüche 1 bis 8, bei der das Nadelgehäuseloch (54) in einem Betätigungsstab (44) ausgebildet ist, der sich an einem am weitesten hinten gelegenen Ende der Betätigungseinheit (34) befindet.

8. Gewebeentnahmevorrichtung (30) nach Anspruch 7, weiterhin umfassend: ein Einführelement (36), das in das Innere des Nadelröhrchens (35) eingeführt ist,
wobei der Betätigungsstab (44) ein Betätigungsstab ist, der das Einführelement (36) in axialer Richtung bezüglich des Nadelröhrchens (35) bewegt.

9. Gewebeentnahmevorrichtung (30) nach Anspruch 6, bei der das Nadelgehäuseloch (54) und die Sperrnut (52) in einem Betätigungsstab (44) ausgebildet sind, der sich an dem am weitesten hinten gelegenen Ende der Betätigungseinheit (32) befindet.

10. Gewebeentnahmevorrichtung (30) nach Anspruch 9, weiterhin umfassend: ein Einführelement (36), das in das Innere des Nadelröhrchens (35) eingesetzt ist,
wobei der Betätigungsstab (44) ein Betätigungsstab ist, der das Einführelement (36) in axialer Richtung bezüglich des Nadelröhrchens (35) bewegt.

11. Gewebeentnahmevorrichtung (30) nach einem der Ansprüche 1 bis 6, bei der das Nadelgehäuseloch (54) in einem Halteelement ausgebildet ist, das lösbar in der Betätigungseinheit (32) angebracht ist.

12. Gewebeentnahmevorrichtung (30) nach Anspruch 6, bei der das Nadelgehäuseloch (54) und die Sperrnut (52) in einem Aufnahmeelement (92) ausgebildet sind, welches in der Betätigungseinheit (32) anbringbar und von dieser lösbar ist.

## Revendications

1. Appareil de récolte de tissu (30), comprenant :
une tube à aiguille (35) présentant une pointe d'aiguille (35b) au niveau de son extrémité avant, la pointe d'aiguille (35b) réalisant la ponction ;
un tube externe (34), dans lequel le tube à aiguille (35) est introduit à l'intérieur et la pointe d'aiguille (35b) du tube à aiguille (35) entre et sort à partir de l'extrémité avant du tube externe, et
une unité de manoeuvre (32) qui est prévue au niveau d'une extrémité de base du tube externe et qui déplace le tube à aiguille dans une direction axiale, **caractérisé par**
un trou de logement d'aiguille (54) agencé dans l'unité de manoeuvre (32) et configuré pour loger et retenir la pointe d'aiguille (35b) du tube à aiguille (35) à l'intérieur du trou de logement d'aiguille (54).

2. Appareil de récolte de tissu (30) selon la revendication 1, dans lequel un élément empêchant la sortie (60) supportant de manière fixe le tube à aiguille (35) logé dans le trou de logement d'aiguille (54) est agencé à l'intérieur du trou de logement d'aiguille (54).

3. Appareil de récolte de tissu (30) selon la revendication 2, dans lequel l'élément empêchant la sortie (60) est un élément de pression pressant le tube à aiguille (35) logé dans le trou de logement d'aiguille (54) vers une surface de paroi latérale du trou de logement d'aiguille (54).

4. Appareil de récolte de tissu (30) selon la revendication 2, dans lequel l'élément empêchant la sortie (60) est un élément convexe tenant le tube à aiguille (35) logé dans le trou de logement d'aiguille (54) entre l'élément convexe et une surface de paroi latérale du trou de logement d'aiguille (54).

5. Appareil de récolte de tissu (30) selon la revendication 2, dans lequel l'élément empêchant la sortie (60) est un élément élastique dans lequel un trou logeant la pointe d'aiguille (35b) est foré par la pointe d'aiguille (35b) durant le logement de la pointe d'aiguille (35b) du tube à aiguille (35) dans le trou de logement d'aiguille (54).

6. Appareil de récolte de tissu (30) selon l'une quelconque des revendications 1 à 5, dans lequel l'unité de manoeuvre (32) présente une rainure de blocage (52) bloquant le tube externe (34) enroulé sous la forme d'une boucle conjointement au tube à aiguille (35), lors de la mise au rebut de l'appareil de récolte de tissu (30).

7. Appareil de récolte de tissu (30) selon l'une quelconque des revendications 1 à 6, dans lequel le trou de logement d'aiguille (54) est formé dans une tige de manoeuvre (44) qui est agencée au niveau d'une extrémité la plus à l'arrière de l'unité de manoeuvre (34).

8. Appareil de récolte de tissu (30) selon la revendication 7, comprenant en outre : un élément d'introduction (36), introduit dans l'intérieur du tube à aiguille (35),
dans lequel la tige de manoeuvre (44) est une tige de manoeuvre déplaçant l'élément d'introduction (36) dans la direction axiale par rapport au tube à aiguille (35).

9. Appareil de récolte de tissu (30) selon la revendication 6, dans lequel le trou de logement d'aiguille (54) et la rainure de blocage (52) sont formés dans une tige de manoeuvre (44) qui est agencée au niveau de l'extrémité la plus à l'arrière de l'unité de manoeuvre (32).

10. Appareil de récolte de tissu (30) selon la revendication 9, comprenant en outre : un élément d'introduction (36), introduit dans l'intérieur du tube à aiguille (35),
dans lequel la tige de manoeuvre (44) est une tige de manoeuvre déplaçant l'élément d'introduction (36) dans la direction axiale par rapport au tube à aiguille (35).

11. Appareil de récolte de tissu (30) selon l'une quelconque des revendications 1 à 6, dans lequel le trou de logement d'aiguille (54) est formé dans un élément de monture pouvant être monté de manière amovible dans l'unité de manoeuvre (32).

12. Appareil de récolte de tissu (30) selon la revendication 6, dans lequel le trou de logement d'aiguille (54) et la rainure de blocage (52) sont formés dans un élément de monture (92) pouvant être attaché à l'unité de manoeuvre (32) et détaché de celle-ci.
